# EUROPEAN PATENT APPLICATION

(11) **EP 3 569 702 A1**
(43) Date of publication of application: **20.11.2019**
(21) Application number: 18203650.9
(22) Date of filing: 31.10.2018
(51) Int. Cl.: C12N 9/04, C07K 14/395, C12N 9/02, C12P 33/00, C12N 1/19

(54) **INCREASE OF GINSENOSIDE PRODUCTION BY IMPROVEMENT OF NADPH-RELATED BIOSYNTHETIC PATHWAY IN YEAST**

(30) Priority: 16.05.2018 KR 20180056159
(71) Applicant: Intelligent Synthetic Biology Center, Daejeon 34141 (KR)
(72) Inventor: KIM, Sun-Chang, 34141 Daejeon (KR); LEE, Ju Young, 18454 Gyeonggi-do (KR); KIM, Jae Eung, 37584 Gyeongsangbuk-do (KR); JANG, In Seung, 48046 Busan (KR); JEGAL, Jong Geon, 44459 Ulsan (KR)
(74) Representative: Gassner, Birgitta

(57) **Abstract**

The present invention relates to yeast with an enhanced ginsenoside-producing ability prepared by changing *Ald2, Ald6, Zwf1,* and *Zms1,* a method of preparing the yeast, and a method for producing ginsenosides using the yeast.

<110>	Intelligent Synthetic Biology Center
	KOREA RESEARCH INSTITUTE OF CHEMICAL TECHNOLOGY

<120>	INCREASE OF GINSENOSIDE PRODUCTION BY IMPROVEMENT OF NADPH-RELATED
BIOSYNTHETIC PATHWAY IN YEAST

<130>	KPA180025-KR

<150>	KR 10-2018-0056159
<151>	2018-05-16

<160>	34

<170>	KoPatentIn 3.0

<210>	1
<211>	1521
<212>	DNA
<213>	Unknown

<220>
<223>	Ald2


<400>	1
atgcctacct tgtatactga tatcgaaatc ccacaattga aaatctcttt aaagcaaccg	60

ctagggttgt ttatcaacaa tgagttttgt ccatcatcag atggaaagac catcgaaact	120

gtgaacccag ctactggcga accgataaca tccttccaag cagctaacga aaaggatgta	180

gacaaagctg tgaaagctgc cagggctgct tttgataacg tttggtcgaa gacatcttct	240

gagcaacgtg gtatttatct ttcaaactta ttaaaactta ttgaggagga gcaagacaca	300

cttgccgcat tagagacttt agacgctgga aagccttacc attcaaatgc caaaggtgat	360

ttggcacaaa ttttacagct taccagatat tttgctgggt ccgctgataa gtttgacaaa	420

ggtgcaacca taccattgac ttttaacaag tttgcatata ctctaaaagt tccttttggc	480

gttgttgctc aaatcgttcc atggaattat cctctagcta tggcttgttg gaaattgcaa	540

ggtgccttag cagccggtaa cacggttatc atcaaacctg ctgagaatac ctctctatct	600

ctactttatt ttgctacttt aattaaaaaa gcaggttttc cacctggtgt tgtcaatatc	660

gttcctggtt atggatcact tgtaggccaa gccctagcat ctcacatgga tatcgacaaa	720

atatctttta cgggaagcac caaggtcggt ggatttgtgt tggaagcttc cggccaatcg	780

aaccttaaag acgttacact agaatgcggt ggtaagtctc ctgctctcgt atttgaagat	840

gcagaccttg ataaggctat cgattggata gcagctggca ttttctacaa ttcaggacag	900

aattgtaccg caaactcaag agtttatgtt caaagttcga tctacgacaa gtttgttgaa	960

aagtttaaag aaactgcaaa gaaggagtgg gatgttgcag gaaaatttga tccgtttgat	1020

gagaaatgca tcgttggtcc agttatatca agtacacagt atgaccgcat caaaagttac	1080

atagaacgtg gtaaaaggga ggaaaagttg gacatgttcc agacctctga atttcctatt	1140

ggtggagcta aaggctactt cattccccca accatcttca ctgatgtccc gcaaacatcg 1200

aaactgttac aggatgagat atttggcccg gttgtggttg ttagcaagtt cacaaattat 1260

gatgacgctc tgaagctggc taatgatact tgctacgggc tcgcctctgc ggtcttcaca 1320

aaagatgtca agaaagcgca catgtttgct cgcgatatta aagcaggaac tgtttggatc 1380

aactcatcta acgatgaaga tgttaccgtt ccttttggcg ggtttaaaat gagtggtatt 1440

ggtagagaac tggggcaaag tggtgttgat acctatcttc aaacaaaagc agttcacata 1500

aatctctctt tggacaacta a 1521

<210> 2
<211> 1503
<212> DNA
<213> Unknown

<220>
<223> Ald6

<400> 2
atgactaagc tacactttga cactgctgaa ccagtcaaga tcacacttcc aaatggtttg 60

acatacgagc aaccaaccgg tctattcatt aacaacaagt ttatgaaagc tcaagacggt 120

aagacctatc ccgtcgaaga tccttccact gaaaacaccg tttgtgaggt ctcttctgcc 180

accactgaag atgttgaata tgctatcgaa tgtgccgacc gtgctttcca cgacactgaa 240

tgggctaccc aagacccaag agaaagaggc cgtctactaa gtaagttggc tgacgaattg 300

gaaagccaaa ttgacttggt ttcttccatt gaagctttgg acaatggtaa aactttggcc 360

ttagcccgtg gggatgttac cattgcaatc aactgtctaa gagatgctgc tgcctatgcc 420

gacaaagtca acggtagaac aatcaacacc ggtgacggct acatgaactt caccacctta 480

gagccaatcg gtgtctgtgg tcaaattatt ccatggaact ttccaataat gatgttggct 540

tggaagatcg ccccagcatt ggccatgggt aacgtctgta tcttgaaacc cgctgctgtc 600

acacctttaa atgccctata ctttgcttct ttatgtaaga aggttggtat tccagctggt 660

gtcgtcaaca tcgttccagg tcctggtaga actgttggtg ctgctttgac caacgaccca 720

agaatcagaa agctggcttt taccggttct acagaagtcg gtaagagtgt tgctgtcgac 780

tcttctgaat ctaacttgaa gaaaatcact ttggaactag gtggtaagtc cgcccatttg 840

gtctttgacg atgctaacat taagaagact ttaccaaatc tagtaaacgg tattttcaag 900

aacgctggtc aaatttgttc ctctggttct agaatttacg ttcaagaagg tatttacgac 960

gaactattgg ctgctttcaa ggcttacttg gaaaccgaaa tcaaagttgg taatccattt 1020

gacaaggcta acttccaagg tgctatcact aaccgtcaac aattcgacac aattatgaac 1080

tacatcgata tcggtaagaa agaaggcgcc aagatcttaa ctggtggcga aaaagttggt 1140

gacaagggtt acttcatcag accaaccgtt ttctacgatg ttaatgaaga catgagaatt 1200

gttaaggaag aaatttttgg accagttgtc actgtcgcaa agttcaagac tttagaagaa 1260

ggtgtcgaaa tggctaacag ctctgaattc ggtctaggtt ctggtatcga aacagaatct 1320

ttgagcacag gtttgaaggt ggccaagatg ttgaaggccg gtaccgtctg gatcaacaca 1380

tacaacgatt ttgactccag agttccattc ggtggtgtta agcaatctgg ttacggtaga 1440

gaaatgggtg aagaagtcta ccatgcatac actgaagtaa aagctgtcag aattaagttg 1500

taa 1503

<210> 3
<211> 1518
<212> DNA
<213> Unknown

<220>
<223> Zwf1

<400> 3
atgagtgaag gccccgtcaa attcgaaaaa aataccgtca tatctgtctt tggtgcgtca 60

ggtgatctgg caaagaagaa gacttttccc gccttatttg ggcttttcag agaaggttac 120

cttgatccat ctaccaagat cttcggttat gcccggtcca aattgtccat ggaggaggac 180

ctgaagtccc gtgtcctacc ccacttgaaa aaacctcacg gtgaagccga tgactctaag 240

gtcgaacagt tcttcaagat ggtcagctac atttcgggaa attacgacac agatgaaggc 300

ttcgacgaat taagaacgca gatcgagaaa ttcgagaaaa gtgccaacgt cgatgtccca 360

caccgtctct tctatctggc cttgccgcca agcgtttttt tgacggtggc caagcagatc 420

aagagtcgtg tgtacgcaga gaatggcatc acccgtgtaa tcgtagagaa acctttcggc 480

cacgacctgg cctctgccag ggagctgcaa aaaaacctgg ggcccctctt taaagaagaa 540

gagttgtaca gaattgacca ttacttgggt aaagagttgg tcaagaatct tttagtcttg 600

aggttcggta accagttttt gaatgcctcg tggaatagag acaacattca aagcgttcag 660

atttcgttta aagagaggtt cggcaccgaa ggccgtggcg gctatttcga ctctataggc 720

ataatcagag acgtgatgca gaaccatctg ttacaaatca tgactctctt gactatggaa 780

agaccggtgt cttttgaccc ggaatctatt cgtgacgaaa aggttaaggt tctaaaggcc 840

gtggccccca tcgacacgga cgacgtcctc ttgggccagt acggtaaatc tgaggacggg 900

tctaagcccg cctacgtgga tgatgacact gtagacaagg actctaaatg tgtcactttt 960

gcagcaatga ctttcaacat cgaaaacgag cgttgggagg gcgtccccat catgatgcgt 1020

gccggtaagg ctttgaatga gtccaaggtg gagatcagac tgcagtacaa agcggtcgca 1080

tcgggtgtct tcaaagacat tccaaataac gaactggtca tcagagtgca gcccgatgcc 1140

gctgtgtacc taaagtttaa tgctaagacc cctggtctgt caaatgctac ccaagtcaca 1200

gatctgaatc taacttacgc aagcaggtac caagactttt ggattccaga ggcttacgag 1260

gtgttgataa gagacgccct actgggtgac cattccaact ttgtcagaga tgacgaattg 1320

gatatcagtt ggggcatatt caccccatta ctgaagcaca tagagcgtcc ggacggtcca 1380

acaccggaaa tttaccccta cggatcaaga ggtccaaagg gattgaagga atatatgcaa 1440

aaacacaagt atgttatgcc cgaaaagcac ccttacgctt ggcccgtgac taagccagaa 1500

gatacgaagg ataattag 1518

<210> 4
<211> 3798
<212> DNA
<213> Unknown

<220>
<223> Zms1

<400> 4
atgtttgtga acggtaatca atctaatttc gctaagcccg ctggtcaagg tattctgccc 60

attcctaaaa aatctcgaat tattaagact gataagccaa gaccgttctt gtgtcccaca 120

tgcactaggg gttttgtcag gcaggagcat ttgaagagac atcagcattc gcatacccgt 180

gagaaaccgt atctttgtat cttttgcggt aggtgttttg ctcgtagaga tttagtgctc 240

aggcatcagc aaaaacttca tgctgctctt gtaggtacgg gggatccacg gcgaatgacg 300

ccagcaccaa attcgacttc ttcttttgcc tccaagcggc gccattccgt ggcggcggat 360

gatccaaccg accttcatat cattaaaata gccggaaata aagagactat tctacccacc 420

ccgaagaacc ttgctggtaa gacatctgaa gaattgaaag aggccgtggt tgccttggcc 480

aaatcaaata atgtagaact tcccgtctcg gccccagtaa tgaacgataa gcgagagaaa 540

actcctccta gtaaggcagg ctccctagga tttcgagagt tcaagttcag cacgaaaggc 600

gtgccagttc actctgcatc aagcgatgct gttatcgaca gggcgaacac tccctcttcc 660

atgcataaga cgaaaagaca tgcgtctttc tctgcatcca gtgcaatgac ttacatgtct 720

agtagcaata gcccccacca ttcaattacc aatttcgagc tcgttgaaga cgctccgcat 780

caagtcggct tttctactcc acaaatgacc gcgaagcagc tcatggaaag cgtgtcagaa 840

ttggatttac ctccgttaac cctggacgaa ccaccgcaag ctatcaagtt taacttaaat 900

ctatttaaca atgacccctc cggacagcaa caacaacaac aacaacaaca gcaaaattcc 960

acctctagta ccatagtgaa cagcaacaat ggaagtacag ttgctacacc tggagtgtat 1020

ctcttaagta gcggtccatc tttaaccgat cttttgacaa tgaactctgc acatgcaggt 1080

gcgggaggat acatgtctag ccaccattcg ccatttgatt tgggctgctt cagtcatgat 1140

aaaccgacag tttctgaatt taaccttccg tcaagcttcc cgaatactat accgtctaat 1200

tctactacgg cttctaatag ttacagtaat ttggcaaatc aaacttatag gcaaatgagc 1260

aatgagcagc cgcttatgtc actatctcct aaaaacccac caacaactgt ttcagattcc 1320

tcttccacga tcaatttcaa tccaggcaca aataatttac tggaaccatc aatggagccc 1380

aatgataagg atagtaatat cgatcctgct gccatagatg acaagtggtt atcagagttt 1440

attaacaact ctgatccaaa atctaccttc aagatcaact tcaatcattt caatgacatt 1500

gggtttattt attctccacc ttcatcaagg tcatctatac caaacaagtc acctccaaac 1560

cattctgcta cctcattaaa tcatgaaaaa gcttctttat cacctcgctt aaacttgagt 1620

ttgaatggaa gcacagattt accaagtaca ccacaaaacc aactaaagga gccttcctat 1680

tctgacccta tttcccatag ttctcataag aggcgtcgtg atagcgtcat gatggactac 1740

gatctatcca attttttcag ctcaaggcaa ttggatattt ccaaggtatt aaacgggaca 1800

gagcaaaata attctcatgt gaacgacgat gttctcactt tgtctttccc cggcgaaact 1860

gattctaatg caacacagaa acagctgcct gttcttactc cttcggattt gttatctccg 1920

ttttctgtcc cttcagtatc tcaagtgctt tttaccaatg agctaaggag tatgatgcta 1980

gccgacaata atatcgattc aggagccttc cccacaacta gtcaattgaa cgattatgtg 2040

acttactata aggaagaatt ccatccattt ttttcattta ttcatcttcc ttctatcata 2100

cctaatatgg acagttatcc cttgttatta tctatctcca tggtcggagc attgtatggg 2160

tttcattcga cgcatgcaaa agtgttagct aatgcagcta gcacccaaat taggaaaagc 2220

ttgaaagtta gtgagaaaaa cccggagacg acagagttat gggttataca gacattagta 2280

ttgctaacgt tctactgtat tttcaataaa aatacagccg tgatcaaggg gatgcatggt 2340

cagttgacga ctattattcg tctcttgaag gcctctcgtt taaatttgcc cctagagtcc 2400

ctatgccagc cgcctattga gagtgatcat attatggaat atgaaaacag tcctcatatg 2460

ttttcaaaaa taagagagca atacaacgcg ccgaatcaaa tgaacaaaaa ctaccaatat 2520

tttgtattgg cgcagtcacg tatcaggact tgccatgcgg tattacttat atctaactta 2580

ttttcttcac tggtaggtgc tgattgctgt tttcattcag tcgatttaaa atgtggtgtt 2640

ccatgctata aagaagaatt atatcagtgc cgaaattccg atgaatggtc ggacctatta 2700

tgtcaataca aaataacgtt agattcgaaa ttttcgttga ttgaattgtc taatggtaac 2760

gaggcatatg aaaattgttt gaggtttctt tctacaggcg atagtttttt ttacggaaat 2820

gctagggttt cgttaagtac atgtctatca ttgttgatat ctatccatga gaaaatactt 2880

attgaaagaa ataacgcaag gatcagtaat aacaacacca atagcaataa cattgagttg 2940

gacgatattg agtggaagat gacttccaga caacggatcg atacaatgtt aaaatactgg 3000

gaaaaccttt atttgaaaaa tggtggcatc ttgacaccta ccgagaatag catgtcaaca 3060

ataaacgcca atccagcaat gaggttaata attccggtat atttgtttgc caaaatgaga 3120

cggtgtttgg acctggcaca tgttattgag aaaatctggt tgaaagattg gtccaatatg 3180

aataaagctt tggaggaagt ttgctatgac atgggttcat tgagggaagc taccgagtat 3240

gcactgaata tggtggatgc gtggacttca ttttttacgt acattaaaca gggcaagcgc 3300

agaattttca atactcctgt atttgcgacc acatgtatgt tcactgcagt attagtgatt 3360

tcggaataca tgaaatgtgt agaggattgg gcacgcgggt acaatgccaa caaccctaac 3420

tcagcattat tggatttttc ggaccgtgtc ttatggctaa aagcagaaag gattttgaga 3480

agattacaaa tgaacttgat accgaaggag tgtgatgtgt tgaaatcgta cactgatttc 3540

ttaagatggc aggacaagga tgccctagat ttgtcagcac taaatgaaga acaagcacaa 3600

agggccatgg acccgaatac cgatataaat gagacaattc aactaattgt agcggcaagt 3660

ctatcctcca aatgtttata tttgggtgtt caaatattgg gtgatgcgcc aatttggcct 3720

ataatattat cgttcgctca tggtttgcaa tcaagagcta tctatagtgt tacgaaaaaa 3780

agaaacacta gaatataa 3798

<210> 5
<211> 527
<212> PRT
<213> Unknown

<220>
<223> tHMG1(S. cerevisiae HMG-CoA reductase)

<400> 5
Met Ala Ala Asp Gln Leu Val Lys Thr Glu Val Thr Lys Lys Ser Phe
1               5                   10                  15

Thr Ala Pro Val Gln Lys Ala Ser Thr Pro Val Leu Thr Asn Lys Thr
            20                  25                  30

Val Ile Ser Gly Ser Lys Val Lys Ser Leu Ser Ser Ala Gln Ser Ser
        35                  40                  45

Ser Ser Gly Pro Ser Ser Ser Ser Glu Glu Asp Asp Ser Arg Asp Ile
    50                  55                  60

Glu Ser Leu Asp Lys Lys Ile Arg Pro Leu Glu Glu Leu Glu Ala Leu
65                  70                  75                  80

Leu Ser Ser Gly Asn Thr Lys Gln Leu Lys Asn Lys Glu Val Ala Ala
                85                  90                  95

Leu Val Ile His Gly Lys Leu Pro Leu Tyr Ala Leu Glu Lys Lys Leu
            100                 105                 110

Gly Asp Thr Thr Arg Ala Val Ala Val Arg Arg Lys Ala Leu Ser Ile
        115                 120                 125

Leu Ala Glu Ala Pro Val Leu Ala Ser Asp Arg Leu Pro Tyr Lys Asn

130                 135                 140

Tyr Asp Tyr Asp Arg Val Phe Gly Ala Cys Cys Glu Asn Val Ile Gly
145                 150                 155                 160

Tyr Met Pro Leu Pro Val Gly Val Ile Gly Pro Leu Val Ile Asp Gly
                165                 170                 175

Thr Ser Tyr His Ile Pro Met Ala Thr Thr Glu Gly Cys Leu Val Ala
            180                 185                 190

Ser Ala Met Arg Gly Cys Lys Ala Ile Asn Ala Gly Gly Gly Ala Thr
        195                 200                 205

Thr Val Leu Thr Lys Asp Gly Met Thr Arg Gly Pro Val Val Arg Phe
    210                 215                 220

Pro Thr Leu Lys Arg Ser Gly Ala Cys Lys Ile Trp Leu Asp Ser Glu
225                 230                 235                 240

Glu Gly Gln Asn Ala Ile Lys Lys Ala Phe Asn Ser Thr Ser Arg Phe
                245                 250                 255

Ala Arg Leu Gln His Ile Gln Thr Cys Leu Ala Gly Asp Leu Leu Phe
            260                 265                 270

Met Arg Phe Arg Thr Thr Thr Gly Asp Ala Met Gly Met Asn Met Ile
        275                 280                 285

Ser Lys Gly Val Glu Tyr Ser Leu Lys Gln Met Val Glu Glu Tyr Gly
    290                 295                 300

Trp Glu Asp Met Glu Val Val Ser Val Ser Gly Asn Tyr Cys Thr Asp
305                 310                 315                 320

Lys Lys Pro Ala Ala Ile Asn Trp Ile Glu Gly Arg Gly Lys Ser Val
                325                 330                 335

Val Ala Glu Ala Thr Ile Pro Gly Asp Val Val Arg Lys Val Leu Lys
            340                 345                 350

Ser Asp Val Ser Ala Leu Val Glu Leu Asn Ile Ala Lys Asn Leu Val
        355                 360                 365

Gly Ser Ala Met Ala Gly Ser Val Gly Gly Phe Asn Ala His Ala Ala
    370                 375                 380

Asn Leu Val Thr Ala Val Phe Leu Ala Leu Gly Gln Asp Pro Ala Gln
385                 390                 395                 400

Asn Val Glu Ser Ser Asn Cys Ile Thr Leu Met Lys Glu Val Asp Gly
                405                 410                 415

Asp Leu Arg Ile Ser Val Ser Met Pro Ser Ile Glu Val Gly Thr Ile
            420                 425                 430

Gly Gly Gly Thr Val Leu Glu Pro Gln Gly Ala Met Leu Asp Leu Leu
        435                 440                 445

Gly Val Arg Gly Pro His Ala Thr Ala Pro Gly Thr Asn Ala Arg Gln
    450                 455                 460

Leu Ala Arg Ile Val Ala Cys Ala Val Leu Ala Gly Glu Leu Ser Leu

465                 470                 475                 480

Cys Ala Ala Leu Ala Ala Gly His Leu Val Gln Ser His Met Thr His
                485                 490                 495

Asn Arg Lys Pro Ala Glu Pro Thr Lys Pro Asn Asn Leu Asp Ala Thr
            500                 505                 510

Asp Ile Asn Arg Leu Lys Asp Gly Ser Val Thr Cys Ile Lys Ser
        515                 520                 525

<210>    6
<211>    545
<212>    PRT
<213>    Unknown

<220>
<223>    PgSE (Panax ginseng, squalene epoxidase)

<400>    6
Met Glu Leu Glu Arg Ser Tyr Arg Glu Asn Asp Glu Tyr Phe Leu Met
1               5                   10                  15

Phe Ala Ala Thr Leu Leu Phe Gly Phe Val Leu Tyr Leu Phe Thr Leu
            20                  25                  30

Arg Arg Arg Arg Arg Arg Arg Glu Lys Lys Gly Gly Ala Gly Ser Met
        35                  40                  45

Glu Ile Ile Asn Gly Ala Tyr Lys Met Thr Ser Ser Ser Glu Val Asn
    50                  55                  60

Gly His Cys Thr Pro Glu Asp Ile Ala Gly Ser Ser Asp Asp Val Ile
65                  70                  75                  80

Ile Val Gly Ala Gly Val Ala Gly Ser Ala Leu Ala Tyr Thr Leu Ala
                85                  90                  95

Lys Asp Gly Arg Arg Val His Val Ile Glu Arg Asp Leu Thr Glu Gln
            100                 105                 110

Asp Arg Ile Val Gly Glu Leu Leu Gln Pro Gly Gly Tyr Leu Lys Leu
        115                 120                 125

Val Glu Leu Gly Leu Glu Asp Cys Val Asn Glu Ile Asp Ala Gln Arg
    130                 135                 140

Val Phe Gly Tyr Ala Leu Tyr Met Asp Gly Lys Asn Thr Arg Leu Ser
145                 150                 155                 160

Tyr Pro Leu Glu Lys Phe His Ala Asp Val Ala Gly Arg Ser Phe His
                165                 170                 175

Asn Gly Arg Phe Ile Gln Arg Met Arg Glu Lys Ala Ala Ser Leu Pro
            180                 185                 190

Asn Val Arg Met Glu Gln Gly Thr Val Thr Ser Leu Val Glu Gln Lys
        195                 200                 205

Gly Thr Val Lys Gly Val Arg Tyr Lys Thr Lys Asn Gly Gln Glu Met
    210                 215                 220

Ser Ala Ala Tyr Ala Pro Leu Thr Ile Val Cys Asp Gly Cys Phe Ser
225                 230                 235                 240

Asn Leu Arg His Ser Leu Cys Asn Pro Lys Val Asp Val Pro Ser Cys
                245                 250                 255

Phe Val Gly Leu Ile Leu Glu Asn Ile Asp Leu Pro His Ile Asn His
            260                 265                 270

Gly His Val Ile Leu Ala Asp Pro Ser Pro Ile Leu Phe Tyr Lys Ile
        275                 280                 285

Ser Ser Thr Glu Ile Arg Cys Leu Val Asp Val Pro Gly Gln Lys Val
    290                 295                 300

Pro Ser Ile Ala Asn Gly Glu Leu Ala His Tyr Leu Lys Thr Ser Val
305                 310                 315                 320

Ala Pro Gln Ile Pro Pro Glu Leu Tyr Lys Ser Phe Ile Ala Ala Ile
                325                 330                 335

Asp Lys Gly Lys Ile Lys Thr Met Pro Asn Arg Ser Met Pro Ala Asp
            340                 345                 350

Pro His Ser Thr Pro Gly Ala Leu Leu Leu Gly Asp Ala Phe Asn Met
        355                 360                 365

Arg His Pro Leu Thr Gly Gly Gly Met Thr Val Ala Leu Ser Asp Ile
    370                 375                 380

Val Leu Ile Arg Asp Leu Leu Arg Pro Leu Arg Asp Leu His Asp Ser
385                 390                 395                 400

Ser Thr Leu Cys Lys Tyr Leu Glu Ser Phe Tyr Thr Leu Arg Lys Pro
                405                 410                 415

Val Ala Ser Thr Ile Asn Thr Leu Ala Gly Ala Leu Tyr Lys Val Phe
            420                 425                 430

Cys Ala Ser Pro Asp Lys Ala Arg Gln Glu Met Arg Asp Ala Cys Phe
        435                 440                 445

Asp Tyr Leu Ser Leu Gly Gly Ile Cys Ser Glu Gly Pro Ile Ala Leu
    450                 455                 460

Leu Ser Gly Leu Asn Pro Arg Pro Met Ser Leu Phe Phe His Phe Phe
465                 470                 475                 480

Ala Val Ala Ile Tyr Gly Val Gly Arg Leu Leu Ile Pro Phe Pro Ser
                485                 490                 495

Pro Arg Lys Met Trp Leu Gly Ala Arg Leu Ile Ser Gly Ala Ser Gly
            500                 505                 510

Ile Ile Phe Pro Ile Ile Lys Ser Glu Gly Val Arg Gln Met Phe Phe
        515                 520                 525

Pro Ala Thr Val Pro Ala Tyr Tyr Arg Ala Pro Pro Ile Thr Lys Lys
    530                 535                 540

Met
545

<210>    7
<211>    769
<212>    PRT
<213>    Unknown

<220>
<223>    PgDS (Panax ginseng, dammarenediol-II synthase)

<400>    7
Met Trp Lys Gln Lys Gly Ala Gln Gly Asn Asp Pro Tyr Leu Tyr Ser
1               5                   10                  15

Thr Asn Asn Phe Val Gly Arg Gln Tyr Trp Glu Phe Gln Pro Asp Ala
            20                  25                  30

Gly Thr Pro Glu Glu Arg Glu Glu Val Glu Lys Ala Arg Lys Asp Tyr
        35                  40                  45

Val Asn Asn Lys Lys Leu His Gly Ile His Pro Cys Ser Asp Met Leu
    50                  55                  60

Met Arg Arg Gln Leu Ile Lys Glu Ser Gly Ile Asp Leu Leu Ser Ile
65                  70                  75                  80

Pro Pro Leu Arg Leu Asp Glu Asn Glu Gln Val Asn Tyr Asp Ala Val
                85                  90                  95

Thr Thr Ala Val Lys Lys Ala Leu Arg Leu Asn Arg Ala Ile Gln Ala
            100                 105                 110

His Asp Gly His Trp Pro Ala Glu Asn Ala Gly Ser Leu Leu Tyr Thr
        115                 120                 125

Pro Pro Leu Ile Ile Ala Leu Tyr Ile Ser Gly Thr Ile Asp Thr Ile
    130                 135                 140

Leu Thr Lys Gln His Lys Lys Glu Leu Ile Arg Phe Val Tyr Asn His
145                 150                 155                 160

Gln Asn Glu Asp Gly Gly Trp Gly Ser Tyr Ile Glu Gly His Ser Thr
                165                 170                 175

Met Ile Gly Ser Val Leu Ser Tyr Val Met Leu Arg Leu Leu Gly Glu
            180                 185                 190

Gly Leu Ala Glu Ser Asp Asp Gly Asn Gly Ala Val Glu Arg Gly Arg
        195                 200                 205

Lys Trp Ile Leu Asp His Gly Gly Ala Ala Gly Ile Pro Ser Trp Gly
    210                 215                 220

Lys Thr Tyr Leu Ala Val Leu Gly Val Tyr Glu Trp Glu Gly Cys Asn
225                 230                 235                 240

Pro Leu Pro Pro Glu Phe Trp Leu Phe Pro Ser Ser Phe Pro Phe His
                245                 250                 255

Pro Ala Lys Met Trp Ile Tyr Cys Arg Cys Thr Tyr Met Pro Met Ser

260                 265                 270

Tyr Leu Tyr Gly Lys Arg Tyr His Gly Pro Ile Thr Asp Leu Val Leu
        275                 280                 285

Ser Leu Arg Gln Glu Ile Tyr Asn Ile Pro Tyr Glu Gln Ile Lys Trp
    290                 295                 300

Asn Gln Gln Arg His Asn Cys Cys Lys Glu Asp Leu Tyr Tyr Pro His
305                 310                 315                 320

Thr Leu Val Gln Asp Leu Val Trp Asp Gly Leu His Tyr Phe Ser Glu
                325                 330                 335

Pro Phe Leu Lys Arg Trp Pro Phe Asn Lys Leu Arg Lys Arg Gly Leu
            340                 345                 350

Lys Arg Val Val Glu Leu Met Arg Tyr Gly Ala Thr Glu Thr Arg Phe
        355                 360                 365

Ile Thr Thr Gly Asn Gly Glu Lys Ala Leu Gln Ile Met Ser Trp Trp
    370                 375                 380

Ala Glu Asp Pro Asn Gly Asp Glu Phe Lys His His Leu Ala Arg Ile
385                 390                 395                 400

Pro Asp Phe Leu Trp Ile Ala Glu Asp Gly Met Thr Val Gln Ser Phe
                405                 410                 415

Gly Ser Gln Leu Trp Asp Cys Ile Leu Ala Thr Gln Ala Ile Ile Ala
            420                 425                 430

Thr Asn Met Val Glu Glu Tyr Gly Asp Ser Leu Lys Lys Ala His Phe
        435                 440                 445

Phe Ile Lys Glu Ser Gln Ile Lys Glu Asn Pro Arg Gly Asp Phe Leu
    450                 455                 460

Lys Met Cys Arg Gln Phe Thr Lys Gly Ala Trp Thr Phe Ser Asp Gln
465                 470                 475                 480

Asp His Gly Cys Val Val Ser Asp Cys Thr Ala Glu Ala Leu Lys Cys
                485                 490                 495

Leu Leu Leu Leu Ser Gln Met Pro Gln Asp Ile Val Gly Glu Lys Pro
            500                 505                 510

Glu Val Glu Arg Leu Tyr Glu Ala Val Asn Val Leu Leu Tyr Leu Gln
        515                 520                 525

Ser Arg Val Ser Gly Gly Phe Ala Val Trp Glu Pro Pro Val Pro Lys
    530                 535                 540

Pro Tyr Leu Glu Met Leu Asn Pro Ser Glu Ile Phe Ala Asp Ile Val
545                 550                 555                 560

Val Glu Arg Glu His Ile Glu Cys Thr Ala Ser Val Ile Lys Gly Leu
                565                 570                 575

Met Ala Phe Lys Cys Leu His Pro Gly His Arg Gln Lys Glu Ile Glu
            580                 585                 590

Asp Ser Val Ala Lys Ala Ile Arg Tyr Leu Glu Arg Asn Gln Met Pro

595                 600                 605

Asp Gly Ser Trp Tyr Gly Phe Trp Gly Ile Cys Phe Leu Tyr Gly Thr
    610                 615                 620

Phe Phe Thr Leu Ser Gly Phe Ala Ser Ala Gly Arg Thr Tyr Asp Asn
625                 630                 635                 640

Ser Glu Ala Val Arg Lys Gly Val Lys Phe Phe Leu Ser Thr Gln Asn
                645                 650                 655

Glu Glu Gly Gly Trp Gly Glu Ser Leu Glu Ser Cys Pro Ser Glu Lys
            660                 665                 670

Phe Thr Pro Leu Lys Gly Asn Arg Thr Asn Leu Val Gln Thr Ser Trp
        675                 680                 685

Ala Met Leu Gly Leu Met Phe Gly Gly Gln Ala Glu Arg Asp Pro Thr
    690                 695                 700

Pro Leu His Arg Ala Ala Lys Leu Leu Ile Asn Ala Gln Met Asp Asn
705                 710                 715                 720

Gly Asp Phe Pro Gln Gln Glu Ile Thr Gly Val Tyr Cys Lys Asn Ser
                725                 730                 735

Met Leu His Tyr Ala Glu Tyr Arg Asn Ile Phe Pro Leu Trp Ala Leu
            740                 745                 750

Gly Glu Tyr Arg Lys Arg Val Trp Leu Pro Lys His Gln Gln Leu Lys
        755                 760                 765

Ile

<210>   8
<211>   482
<212>   PRT
<213>   Unknown

<220>
<223>   PgPPDS (Panax ginseng cytochrome P450 CYP716A47)

<400>   8
Met Val Leu Phe Phe Ser Leu Ser Leu Leu Leu Leu Pro Leu Leu Leu
1               5                   10                  15

Leu Phe Ala Tyr Phe Ser Tyr Thr Lys Arg Ile Pro Gln Lys Glu Asn
            20                  25                  30

Asp Ser Lys Ala Pro Leu Pro Pro Gly Gln Thr Gly Trp Pro Leu Ile
        35                  40                  45

Gly Glu Thr Leu Asn Tyr Leu Ser Cys Val Lys Ser Gly Val Ser Glu
    50                  55                  60

Asn Phe Val Lys Tyr Arg Lys Glu Lys Tyr Ser Pro Lys Val Phe Arg
65                  70                  75                  80

Thr Ser Leu Leu Gly Glu Pro Met Ala Ile Leu Cys Gly Pro Glu Gly
                85                  90                  95

Asn Lys Phe Leu Tyr Ser Thr Glu Lys Lys Leu Val Gln Val Trp Phe
            100                 105                 110

Pro Ser Ser Val Glu Lys Met Phe Pro Arg Ser His Gly Glu Ser Asn
        115                 120                 125

Ala Asp Asn Phe Ser Lys Val Arg Gly Lys Met Met Phe Leu Leu Lys
    130                 135                 140

Val Asp Gly Met Lys Lys Tyr Val Gly Leu Met Asp Arg Val Met Lys
145                 150                 155                 160

Gln Phe Leu Glu Thr Asp Trp Asn Arg Gln Gln Gln Ile Asn Val His
                165                 170                 175

Asn Thr Val Lys Lys Tyr Thr Val Thr Met Ser Cys Arg Val Phe Met
            180                 185                 190

Ser Ile Asp Asp Glu Glu Gln Val Thr Arg Leu Gly Ser Ser Ile Gln
        195                 200                 205

Asn Ile Glu Ala Gly Leu Leu Ala Val Pro Ile Asn Ile Pro Gly Thr
    210                 215                 220

Ala Met Asn Arg Ala Ile Lys Thr Val Lys Leu Leu Thr Arg Glu Val
225                 230                 235                 240

Glu Ala Val Ile Lys Gln Arg Lys Val Asp Leu Leu Glu Asn Lys Gln
                245                 250                 255

Ala Ser Gln Pro Gln Asp Leu Leu Ser His Leu Leu Leu Thr Ala Asn
            260                 265                 270

Gln Asp Gly Gln Phe Leu Ser Glu Ser Asp Ile Ala Ser His Leu Ile
        275                 280                 285

Gly Leu Met Gln Gly Gly Tyr Thr Thr Leu Asn Gly Thr Ile Thr Phe
    290                 295                 300

Val Leu Asn Tyr Leu Ala Glu Phe Pro Asp Val Tyr Asn Gln Val Leu
305                 310                 315                 320

Lys Glu Gln Val Glu Ile Ala Asn Ser Lys His Pro Lys Glu Leu Leu
                325                 330                 335

Asn Trp Glu Asp Leu Arg Lys Met Lys Tyr Ser Trp Asn Val Ala Gln
            340                 345                 350

Glu Val Leu Arg Ile Ile Pro Pro Gly Val Gly Thr Phe Arg Glu Ala
        355                 360                 365

Ile Thr Asp Phe Thr Tyr Ala Gly Tyr Leu Ile Pro Lys Gly Trp Lys
    370                 375                 380

Met His Leu Ile Pro His Asp Thr His Lys Asn Pro Thr Tyr Phe Pro
385                 390                 395                 400

Ser Pro Glu Lys Phe Asp Pro Thr Arg Phe Glu Gly Asn Gly Pro Ala
                405                 410                 415

Pro Tyr Thr Phe Thr Pro Phe Gly Gly Gly Pro Arg Met Cys Pro Gly
            420                 425                 430

Ile Glu Tyr Ala Arg Leu Val Ile Leu Ile Phe Met His Asn Val Val
        435                 440                 445

Thr Asn Phe Arg Trp Glu Lys Leu Ile Pro Asn Glu Lys Ile Leu Thr
    450                 455                 460

Asp Pro Ile Pro Arg Phe Ala His Gly Leu Pro Ile His Leu His Pro
465                 470                 475                 480

His Asn

<210>   9
<211>   709
<212>   PRT
<213>   Unknown

<220>
<223>   PgCPR (Panax NADPH-cytochrome P450 reductase)

<400>   9
Met Leu Lys Val Ser Pro Phe Asp Leu Met Thr Glu Ile Leu Arg Gly
  1               5                  10                  15

Gly Ser Ile Asp Pro Pro Asn Ser Ser Val Ser Ala Ala Gly Ala Ser
             20                  25                  30

Met Gln Pro Ser Leu Ala Met Leu Val Val Asn Arg Glu Leu Leu Met
         35                  40                  45

Leu Leu Thr Thr Ser Val Ala Val Leu Ile Gly Cys Val Val Val Leu
    50                  55                  60

Val Trp Arg Lys Ser Ser Ser Gln Lys His Ala Lys Ser Phe Glu Ala
 65                  70                  75                  80

Pro Lys Leu Leu Ile Pro Lys Ile Glu Pro Glu Glu Val Val Asp Asp
                 85                  90                  95

Gly Lys Lys Lys Val Thr Ile Phe Phe Gly Thr Gln Thr Gly Thr Ala
            100                 105                 110

Glu Gly Phe Ala Lys Ala Leu Ala Glu Glu Ala Lys Ala Arg Tyr Glu
        115                 120                 125

Lys Ala Ile Phe Lys Val Ile Asp Leu Asp Asp Tyr Ala Pro Glu Asp
    130                 135                 140

Asp Asp Tyr Glu Thr Lys Leu Lys Lys Glu Ser Leu Ala Phe Phe Phe
145                 150                 155                 160

Leu Ala Thr Tyr Gly Asp Gly Glu Pro Thr Asp Asn Ala Ala Arg Phe
                165                 170                 175

Tyr Lys Trp Phe Thr Glu Gly Lys Glu Lys Arg Glu Trp Leu Asn Asn
            180                 185                 190

Leu Gln Tyr Gly Val Phe Gly Leu Gly Asn Arg Gln Tyr Glu His Phe
        195                 200                 205

Asn Lys Ile Ala Lys Val Val Asp Asp Gly Leu Ala Glu Gln Gly Ala
    210                 215                 220

Lys Arg Leu Val Pro Val Gly Met Gly Asp Asp Asp Gln Cys Ile Glu
225                 230                 235                 240

Asp Asp Phe Thr Ala Trp Arg Glu Leu Val Trp Pro Glu Leu Asp Gln
                245                 250                 255

Leu Leu Leu Asp Glu Glu Asp Thr Ala Ala Ala Thr Pro Tyr Thr Ala
            260                 265                 270

Ala Val Leu Glu Tyr Arg Val Val Phe His Asp Arg Thr Asp Ser Ser
        275                 280                 285

Thr Leu Leu Asn Gly Thr Thr Ser Val Ser Asn Gly His Ala Phe Tyr
    290                 295                 300

Asp Ala Gln His Pro Cys Arg Ala Asn Val Ala Val Lys Arg Glu Leu
305                 310                 315                 320

His Thr Leu Glu Ser Asp Arg Ser Cys Thr His Leu Glu Phe Asp Ile
                325                 330                 335

Ser Ser Thr Gly Leu Ala Tyr Glu Thr Gly Asp His Val Gly Val Tyr
            340                 345                 350

Thr Glu Asn Leu Ile Glu Ile Val Glu Glu Ala Glu Arg Leu Leu Ala
        355                 360                 365

Ile Ser Pro Asp Thr Tyr Phe Ser Ile His Thr Glu Lys Glu Asp Gly
    370                 375                 380

Ser Pro Val Ser Gly Ser Ser Leu Gln Pro Pro Phe Pro Pro Cys Thr
385                 390                 395                 400

Leu Arg Glu Ala Leu Arg Arg Tyr Ala Asp Leu Leu Ser Ser Pro Lys
                405                 410                 415

Lys Ser Ala Leu Leu Ala Leu Ala Ala His Ala Ser Asp Pro Ser Glu
            420                 425                 430

Ala Asp Arg Leu Arg Phe Leu Ala Ser Pro Ala Gly Lys Asp Glu Tyr
        435                 440                 445

Ala Gln Trp Ile Val Ala Asn Gln Arg Ser Leu Leu Glu Val Leu Ala
    450                 455                 460

Glu Phe Pro Ser Ala Lys Pro Pro Leu Gly Val Phe Phe Ala Ser Val
465                 470                 475                 480

Ala Pro Arg Leu Gln Pro Arg Tyr Tyr Ser Ile Ser Ser Ser Pro Arg
                485                 490                 495

Met Ala Pro Ser Arg Ile His Val Thr Cys Ala Leu Val Phe Glu Arg
            500                 505                 510

Thr Pro Ala Gly Arg Ile His Lys Gly Val Cys Ser Thr Trp Met Lys
        515                 520                 525

Asn Ala Val Ser Leu Glu Glu Gly Asn Asp Cys Ser Arg Ala Pro Ile
    530                 535                 540

Phe Val Arg Gln Ser Asn Phe Lys Leu Pro Ser Asp Ser Arg Met Pro
545                 550                 555                 560

Ile Ile Met Ile Gly Pro Gly Thr Gly Leu Ala Pro Phe Arg Gly Phe
                565                 570                 575

Leu Gln Glu Arg Leu Ala Leu Lys Glu Ala Gly Ala Glu Leu Gly Pro
            580                 585                 590

Ala Val Leu Tyr Phe Gly Cys Arg Asn Arg Lys Leu Asp Phe Ile Tyr
        595                 600                 605

Glu Asp Glu Leu Asn Asn Phe Val Glu Ser Gly Ala Ile Ser Glu Met
    610                 615                 620

Val Val Ala Phe Ser Arg Glu Gly Pro Thr Lys Glu Tyr Val Gln His
625                 630                 635                 640

Lys Met Ser Gln Lys Ala Ser Glu Ile Trp Asn Met Ile Ser Glu Gly
                645                 650                 655

Ala Tyr Ile Tyr Val Cys Gly Asp Ala Lys Gly Met Ala Arg Asp Val
            660                 665                 670

His Arg Thr Leu His Thr Ile Ala Gln Glu Gln Gly Ala Leu Asp Ser
        675                 680                 685

Ser Lys Ala Glu Ser Leu Val Lys Asn Leu Gln Met Thr Gly Arg Tyr
    690                 695                 700

Leu Arg Asp Val Trp
705


<210>    10
<211>    70
<212>    DNA
<213>    Artificial Sequence

<220>
<223>    Del_Ald2_F


<400>    10
ttacattgca tgtccatcaa aaacaatcgt gaaaataagc caaaagaaaa ccagtcacga          60

cgttgtaaaa                                                                  70


<210>    11
<211>    70
<212>    DNA
<213>    Artificial Sequence

<220>
<223>    Del_Ald2_R


<400>    11
ctgcaacatc ccactccttc tttgcagttt ctttaaactt ttcaacaaac aggtttcccg          60

actggaaagc                                                                  70

<210>    12
<211>    25
<212>    DNA
<213>    Artificial Sequence

<220>
<223>    Ald2_conf_F


<400>    12
ttacattgca tgtccatcaa aaaca                                          25


<210>    13
<211>    20
<212>    DNA
<213>    Artificial Sequence

<220>
<223>    Ald2_conf_R


<400>    13
ctgcaacatc ccactccttc                                                20


<210>    14
<211>    70
<212>    DNA
<213>    Artificial Sequence

<220>
<223>    Del_Gdh1_F


<400>    14
actatcgcat tattctaata taacagttag gagaccaaaa agaaaaagaa ccagtcacga    60

cgttgtaaaa                                                           70


<210>    15
<211>    70
<212>    DNA
<213>    Artificial Sequence

<220>
<223>    Del_Gdh1_R


<400>    15
gacggcaata gcttctggag tggaacccat gttggaacct tcggcaataa aggtttcccg    60

actggaaagc                                                           70


<210>    16
<211>    27
<212>    DNA
<213>    Artificial Sequence

<220>

<223>    Gdh1_conf_F

<400>    16
cagttaggag accaaaaaga aaaagaa                                          27

<210>    17
<211>    20
<212>    DNA
<213>    Artificial Sequence

<220>
<223>    Gdh1_conf_R

<400>    17
gacggcaata gcttctggag                                                  20

<210>    18
<211>    4226
<212>    DNA
<213>    Unknown

<220>
<223>    pUC57-URA3Myc

<400>    18
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgaatt cgagctcggt acctcgcgaa     420

tgcatctaga tatcggatcc cgacctggag ctgtgcggcc gttctgagca aaagctcatt     480

tctgaagagg acttgaatgg agaacagaaa ttgatcagtg aggaagacct caacggtgag     540

cagaagttaa tatccgagga ggatcttaat agtagcggcc gtactagcgg atttccccgc     600

ggccgctaca atttcctgat gcggtatttt ctccttacgc atctgtgcgg tatttcacac     660

cgcatagggt aataactgat ataattaaat tgaagctcta atttgtgagt ttagtataca     720

tgcatttact tataatacag ttttttagtt ttgctggccg catcttctca aatatgcttc     780

ccagcctgct tttctgtaac gttcaccctc taccttagca tcccttccct ttgcaaatag     840

tcctcttcca acaataataa tgtcagatcc tgtagagacc acatcatcca cggttctata     900

ctgttgaccc aatgcgtctc ccttgtcatc taaacccaca ccgggtgtca taatcaacca     960

atcgtaacct tcatctcttc cacccatgtc tctttgagca ataaagccga taacaaaatc    1020

tttgtcgctc ttcgcaatgt caacagtacc cttagtatat tctccagtag atagggagcc 1080

cttgcatgac aattctgcta acatcaaaag gcctctaggt tcctttgtta cttcttctgc 1140

cgcctgcttc aaaccgctaa caatacctgg gcccaccaca ccgtgtgcat tcgtaatgtc 1200

tgcccattct gctattctgt atacacccgc agagtactgc aatttgactg tattaccaat 1260

gtcagcaaat tttctgtctt cgaagagtaa aaaattgtac ttggcggata atgcctttag 1320

cggcttaact gtgccctcca tggaaaaatc agtcaagata tccacatgtg tttttagtaa 1380

acaaattttg ggacctaatg cttcaactaa ctccagtaat tccttggtgg tacgaacatc 1440

caatgaagca cacaagtttg tttgcttttc gtgcatgata ttaaatagct tggcagcaac 1500

aggactagga tgagtagcag cacgttcctt atatgtagct ttcgacatga tttatcttcg 1560

tttcctgcag gtttttgttc tgtgcagttg ggttaagaat actgggcaat ttcatgtttc 1620

ttcaacacta catatgcgta tatataccaa tctaagtctg tgctccttcc ttcgttcttc 1680

cttctgttcg gagattaccg aatcaaaaaa atttcaagga aaccgaaatc aaaaaaaaga 1740

ataaaaaaaa aatgatgaat tgaaaaggtg gtatggtgca ctctcaggcg gccgcgaatt 1800

agagctgtgc ggccgttctg agcaaaagct catttctgaa gaggacttga atggagaaca 1860

gaaattgatc agtgaggaag acctcaacgg tgagcagaag ttaatatccg aggaggatct 1920

taatagtagc ggccgtacta gcgggggccc cggagacggg cccgtcgact gcagaggcct 1980

gcatgcaagc ttggcgtaat catggtcata gctgtttcct gtgtgaaatt gttatccgct 2040

cacaattcca cacaacatac gagccggaag cataaagtgt aaagcctggg gtgcctaatg 2100

agtgagctaa ctcacattaa ttgcgttgcg ctcactgccc gctttccagt cgggaaacct 2160

gtcgtgccag ctgcattaat gaatcggcca acgcgcgggg agaggcggtt tgcgtattgg 2220

gcgctcttcc gcttcctcgc tcactgactc gctgcgctcg gtcgttcggc tgcggcgagc 2280

ggtatcagct cactcaaagg cggtaatacg gttatccaca gaatcagggg ataacgcagg 2340

aaagaacatg tgagcaaaag gccagcaaaa ggccaggaac cgtaaaaagg ccgcgttgct 2400

ggcgtttttc cataggctcc gcccccctga cgagcatcac aaaaatcgac gctcaagtca 2460

gaggtggcga aacccgacag gactataaag ataccaggcg tttccccctg gaagctccct 2520

cgtgcgctct cctgttccga ccctgccgct taccggatac ctgtccgcct ttctcccttc 2580

gggaagcgtg gcgctttctc atagctcacg ctgtaggtat ctcagttcgg tgtaggtcgt 2640

tcgctccaag ctgggctgtg tgcacgaacc ccccgttcag cccgaccgct gcgccttatc 2700

cggtaactat cgtcttgagt ccaacccggt aagacacgac ttatcgccac tggcagcagc 2760

cactggtaac aggattagca gagcgaggta tgtaggcggt gctacagagt tcttgaagtg 2820

gtggcctaac tacggctaca ctagaagaac agtatttggt atctgcgctc tgctgaagcc 2880

agttaccttc ggaaaaagag ttggtagctc ttgatccggc aaacaaacca ccgctggtag 2940

cggtggtttt tttgtttgca agcagcagat tacgcgcaga aaaaaaggat ctcaagaaga 3000

tcctttgatc ttttctacgg ggtctgacgc tcagtggaac gaaaactcac gttaagggat 3060

tttggtcatg agattatcaa aaaggatctt cacctagatc cttttaaatt aaaaatgaag 3120

ttttaaatca atctaaagta tatatgagta aacttggtct gacagttacc aatgcttaat 3180

cagtgaggca cctatctcag cgatctgtct atttcgttca tccatagttg cctgactccc 3240

cgtcgtgtag ataactacga tacgggaggg cttaccatct ggccccagtg ctgcaatgat 3300

accgcgagac ccacgctcac cggctccaga tttatcagca ataaaccagc cagccggaag 3360

ggccgagcgc agaagtggtc ctgcaacttt atccgcctcc atccagtcta ttaattgttg 3420

ccgggaagct agagtaagta gttcgccagt taatagtttg cgcaacgttg ttgccattgc 3480

tacaggcatc gtggtgtcac gctcgtcgtt tggtatggct tcattcagct ccggttccca 3540

acgatcaagg cgagttacat gatcccccat gttgtgcaaa aaagcggtta gctccttcgg 3600

tcctccgatc gttgtcagaa gtaagttggc cgcagtgtta tcactcatgg ttatggcagc 3660

actgcataat tctcttactg tcatgccatc cgtaagatgc ttttctgtga ctggtgagta 3720

ctcaaccaag tcattctgag aatagtgtat gcggcgaccg agttgctctt gcccggcgtc 3780

aatacgggat aataccgcgc cacatagcag aactttaaaa gtgctcatca ttggaaaacg 3840

ttcttcgggg cgaaaactct caaggatctt accgctgttg agatccagtt cgatgtaacc 3900

cactcgtgca cccaactgat cttcagcatc ttttactttc accagcgttt ctgggtgagc 3960

aaaaacagga aggcaaaatg ccgcaaaaaa gggaataagg gcgacacgga aatgttgaat 4020

actcatactc ttcctttttc aatattattg aagcatttat cagggttatt gtctcatgag 4080

cggatacata tttgaatgta tttagaaaaa taaacaaata ggggttccgc gcacatttcc 4140

ccgaaaagtg ccacctgacg tctaagaaac cattattatc atgacattaa cctataaaaa 4200

taggcgtatc acgaggccct ttcgtc 4226

<210> 19
<211> 27
<212> DNA
<213> Artificial Sequence

<220>
<223> Gnd1_F

<400> 19
ggaattcatg tctgctgatt tcggttt 27

<210> 20
<211> 34
<212> DNA

<213>    Artificial Sequence

<220>
<223>    Gnd1_R


<400>    20
ccgctcgagt taagcttggt atgtagagga agaa                                    34


<210>    21
<211>    34
<212>    DNA
<213>    Artificial Sequence

<220>
<223>    Gdh2_F


<400>    21
gctctagaat gctttttgat aacaaaaatc gcgg                                    34


<210>    22
<211>    29
<212>    DNA
<213>    Artificial Sequence

<220>
<223>    Gdh2_R


<400>    22
tcccccgggt caagcacttg cctccgctt                                          29


<210>    23
<211>    34
<212>    DNA
<213>    Artificial Sequence

<220>
<223>    Ald6_F


<400>    23
cgggatccat gactaagcta cactttgaca ctgc                                    34


<210>    24
<211>    41
<212>    DNA
<213>    Artificial Sequence

<220>
<223>    Ald6_R


<400>    24
ccgctcgagt tacaacttaa ttctgacagc ttttacttca g                            41


<210>    25

<211>    27
<212>    DNA
<213>    Artificial Sequence

<220>
<223>    Zwf1_F


<400>    25
ggaattcatg agtgaaggcc ccgtcaa                                          27


<210>    26
<211>    34
<212>    DNA
<213>    Artificial Sequence

<220>
<223>    Zwf1_R


<400>    26
ccgctcgagc taattatcct tcgtatcttc tggc                                  34


<210>    27
<211>    29
<212>    DNA
<213>    Artificial Sequence

<220>
<223>    Stb5_F


<400>    27
ggaattcatg gatggtccca attttgcac                                        29


<210>    28
<211>    38
<212>    DNA
<213>    Artificial Sequence

<220>
<223>    Stb5_R


<400>    28
acgcgtcgac tcatacaagt ttatcaaccc aagagacg                              38


<210>    29
<211>    5778
<212>    DNA
<213>    Unknown

<220>
<223>    p416_GPD vector


<400>    29
gacgaaaggg cctcgtgata cgcctatttt tataggttaa tgtcatgata ataatggttt      60

cttaggacgg atcgcttgcc tgtaacttac acgcgcctcg tatcttttaa tgatggaata 120

atttgggaat ttactctgtg tttatttatt tttatgtttt gtatttggat tttagaaagt 180

aaataaagaa ggtagaagag ttacggaatg aagaaaaaaa aataaacaaa ggtttaaaaa 240

atttcaacaa aaagcgtact ttacatatat atttattaga caagaaaagc agattaaata 300

gatatacatt cgattaacga taagtaaaat gtaaaatcac aggattttcg tgtgtggtct 360

tctacacaga caagatgaaa caattcggca ttaatacctg agagcaggaa gagcaagata 420

aaaggtagta tttgttggcg atccccctag agtcttttac atcttcggaa aacaaaaact 480

attttttctt taatttcttt ttttactttc tatttttaat ttatatattt atattaaaaa 540

atttaaatta taattatttt tatagcacgt gatgaaaagg acccaggtgg cacttttcgg 600

ggaaatgtgc gcggaacccc tatttgttta tttttctaaa tacattcaaa tatgtatccg 660

ctcatgagac aataaccctg ataaatgctt caataatatt gaaaaaggaa gagtatgagt 720

attcaacatt tccgtgtcgc ccttattccc ttttttgcgg cattttgcct tcctgttttt 780

gctcacccag aaacgctggt gaaagtaaaa gatgctgaag atcagttggg tgcacgagtg 840

ggttacatcg aactggatct caacagcggt aagatccttg agagttttcg ccccgaagaa 900

cgttttccaa tgatgagcac ttttaaagtt ctgctatgtg gcgcggtatt atcccgtatt 960

gacgccgggc aagagcaact cggtcgccgc atacactatt ctcagaatga cttggttgag 1020

tactcaccag tcacagaaaa gcatcttacg gatggcatga cagtaagaga attatgcagt 1080

gctgccataa ccatgagtga taacactgcg gccaacttac ttctgacaac gatcggagga 1140

ccgaaggagc taaccgcttt tttgcacaac atgggggatc atgtaactcg ccttgatcgt 1200

tgggaaccgg agctgaatga agccatacca aacgacgagc gtgacaccac gatgcctgta 1260

gcaatggcaa caacgttgcg caaactatta actggcgaac tacttactct agcttcccgg 1320

caacaattaa tagactggat ggaggcggat aaagttgcag gaccacttct gcgctcggcc 1380

cttccggctg gctggtttat tgctgataaa tctggagccg gtgagcgtgg gtctcgcggt 1440

atcattgcag cactggggcc agatggtaag ccctcccgta tcgtagttat ctacacgacg 1500

gggagtcagg caactatgga tgaacgaaat agacagatcg ctgagatagg tgcctcactg 1560

attaagcatt ggtaactgtc agaccaagtt tactcatata tactttagat tgatttaaaa 1620

cttcattttt aatttaaaag gatctaggtg aagatccttt ttgataatct catgaccaaa 1680

atcccttaac gtgagttttc gttccactga gcgtcagacc ccgtagaaaa gatcaaagga 1740

tcttcttgag atcctttttt tctgcgcgta atctgctgct tgcaaacaaa aaaaccaccg 1800

ctaccagcgg tggtttgttt gccggatcaa gagctaccaa ctctttttcc gaaggtaact 1860

ggcttcagca gagcgcagat accaaatact gtccttctag tgtagccgta gttaggccac 1920

cacttcaaga actctgtagc accgcctaca tacctcgctc tgctaatcct gttaccagtg 1980

gctgctgcca gtggcgataa gtcgtgtctt accgggttgg actcaagacg atagttaccg 2040

gataaggcgc agcggtcggg ctgaacgggg ggttcgtgca cacagcccag cttggagcga 2100

acgacctaca ccgaactgag atacctacag cgtgagctat gagaaagcgc cacgcttccc 2160

gaagggagaa aggcggacag gtatccggta agcggcaggg tcggaacagg agagcgcacg 2220

agggagcttc cagggggaaa cgcctggtat ctttatagtc ctgtcgggtt tcgccacctc 2280

tgacttgagc gtcgattttt gtgatgctcg tcaggggggc ggagcctatg gaaaaacgcc 2340

agcaacgcgg cctttttacg gttcctggcc ttttgctggc cttttgctca catgttcttt 2400

cctgcgttat cccctgattc tgtggataac cgtattaccg cctttgagtg agctgatacc 2460

gctcgccgca gccgaacgac cgagcgcagc gagtcagtga gcgaggaagc ggaagagcgc 2520

ccaatacgca aaccgcctct ccccgcgcgt tggccgattc attaatgcag ctggcacgac 2580

aggtttcccg actggaaagc gggcagtgag cgcaacgcaa ttaatgtgag ttacctcact 2640

cattaggcac cccaggcttt acactttatg cttccggctc ctatgttgtg tggaattgtg 2700

agcggataac aatttcacac aggaaacagc tatgaccatg attacgccaa gcgcgcaatt 2760

aaccctcact aaagggaaca aaagctggag ctcagtttat cattatcaat actcgccatt 2820

tcaaagaata cgtaaataat taatagtagt gattttccta actttattta gtcaaaaaat 2880

tagcctttta attctgctgt aacccgtaca tgcccaaaat agggggcggg ttacacagaa 2940

tatataacat cgtaggtgtc tgggtgaaca gtttattcct ggcatccact aaatataatg 3000

gagcccgctt tttaagctgg catccagaaa aaaaaagaat cccagcacca aaatattgtt 3060

ttcttcacca accatcagtt cataggtcca ttctcttagc gcaactacag agaacagggg 3120

cacaaacagg caaaaaacgg gcacaacctc aatggagtga tgcaacctgc ctggagtaaa 3180

tgatgacaca aggcaattga cccacgcatg tatctatctc attttcttac accttctatt 3240

accttctgct ctctctgatt tggaaaaagc tgaaaaaaaa ggttgaaacc agttccctga 3300

aattattccc ctacttgact aataagtata taaagacggt aggtattgat tgtaattctg 3360

taaatctatt tcttaaactt cttaaattct acttttatag ttagtctttt ttttagtttt 3420

aaaacaccag aacttagttt cgacggattc tagaactagt ggatcccccg ggctgcagga 3480

attcgatatc aagcttatcg ataccgtcga cctcgagtca tgtaattagt tatgtcacgc 3540

ttacattcac gccctccccc cacatccgct ctaaccgaaa aggaaggagt tagacaacct 3600

gaagtctagg tccctattta tttttttata gttatgttag tattaagaac gttatttata 3660

tttcaaattt ttcttttttt tctgtacaga cgcgtgtacg catgtaacat tatactgaaa 3720

accttgcttg agaaggtttt gggacgctcg aaggctttaa tttgcggccg gtacccaatt 3780

cgccctatag tgagtcgtat tacgcgcgct cactggccgt cgttttacaa cgtcgtgact 3840

gggaaaaccc tggcgttacc caacttaatc gccttgcagc acatccccct ttcgccagct 3900

ggcgtaatag cgaagaggcc cgcaccgatc gcccttccca acagttgcgc agcctgaatg 3960

gcgaatggcg cgacgcgccc tgtagcggcg cattaagcgc ggcgggtgtg gtggttacgc 4020

gcagcgtgac cgctacactt gccagcgccc tagcgcccgc tcctttcgct ttcttccctt 4080

cctttctcgc cacgttcgcc ggctttcccc gtcaagctct aaatcggggg ctccctttag 4140

ggttccgatt tagtgcttta cggcacctcg accccaaaaa acttgattag ggtgatggtt 4200

cacgtagtgg gccatcgccc tgatagacgg tttttcgccc tttgacgttg gagtccacgt 4260

tctttaatag tggactcttg ttccaaactg gaacaacact caaccctatc tcggtctatt 4320

cttttgattt ataagggatt ttgccgattt cggcctattg gttaaaaaat gagctgattt 4380

aacaaaaatt taacgcgaat tttaacaaaa tattaacgtt tacaatttcc tgatgcggta 4440

ttttctcctt acgcatctgt gcggtatttc acaccgcata gggtaataac tgatataatt 4500

aaattgaagc tctaatttgt gagtttagta tacatgcatt tacttataat acagtttttt 4560

agttttgctg gccgcatctt ctcaaatatg cttcccagcc tgcttttctg taacgttcac 4620

cctctacctt agcatccctt ccctttgcaa atagtcctct tccaacaata ataatgtcag 4680

atcctgtaga gaccacatca tccacggttc tatactgttg acccaatgcg tctcccttgt 4740

catctaaacc cacaccgggt gtcataatca accaatcgta accttcatct cttccaccca 4800

tgtctctttg agcaataaag ccgataacaa aatctttgtc gctcttcgca atgtcaacag 4860

tacccttagt atattctcca gtagataggg agcccttgca tgacaattct gctaacatca 4920

aaaggcctct aggttccttt gttacttctt ctgccgcctg cttcaaaccg ctaacaatac 4980

ctgggcccac cacaccgtgt gcattcgtaa tgtctgccca ttctgctatt ctgtatacac 5040

ccgcagagta ctgcaatttg actgtattac caatgtcagc aaattttctg tcttcgaaga 5100

gtaaaaaatt gtacttggcg gataatgcct ttagcggctt aactgtgccc tccatggaaa 5160

aatcagtcaa gatatccaca tgtgttttta gtaaacaaat tttgggacct aatgcttcaa 5220

ctaactccag taattccttg gtggtacgaa catccaatga agcacacaag tttgtttgct 5280

tttcgtgcat gatattaaat agcttggcag caacaggact aggatgagta gcagcacgtt 5340

ccttatatgt agctttcgac atgatttatc ttcgtttcct gcaggttttt gttctgtgca 5400

gttgggttaa gaatactggg caatttcatg tttcttcaac actacatatg cgtatatata 5460

ccaatctaag tctgtgctcc ttccttcgtt cttccttctg ttcggagatt accgaatcaa 5520

aaaaatttca aagaaaccga aatcaaaaaa aagaataaaa aaaaaatgat gaattgaatt 5580

gaaaagctgt ggtatggtgc actctcagta caatctgctc tgatgccgca tagttaagcc 5640

agccccgaca cccgccaaca cccgctgacg cgccctgacg ggcttgtctg ctcccggcat 5700

ccgcttacag acaagctgtg accgtctccg ggagctgcat gtgtcagagg ttttcaccgt 5760

catcaccgaa acgcgcga 5778

<210> 30
<211> 5190
<212> DNA
<213> Unknown

<220>
<223> pUC57GPD-URA3Myc

<400> 30
gacgaaaggg cctcgtgata cgcctatttt tataggttaa tgtcatgata ataatggttt 60

cttagacgtc aggtggcact tttcggggaa atgtgcgcgg aacccctatt tgtttatttt 120

tctaaataca ttcaaatatg tatccgctca tgagacaata accctgataa atgcttcaat 180

aatattgaaa aaggaagagt atgagtattc aacatttccg tgtcgccctt attccctttt 240

ttgcggcatt ttgccttcct gtttttgctc acccagaaac gctggtgaaa gtaaaagatg 300

ctgaagatca gttgggtgca cgagtgggtt acatcgaact ggatctcaac agcggtaaga 360

tccttgagag ttttcgcccc gaagaacgtt ttccaatgat gagcactttt aaagttctgc 420

tatgtggcgc ggtattatcc cgtattgacg ccgggcaaga gcaactcggt cgccgcatac 480

actattctca gaatgacttg gttgagtact caccagtcac agaaaagcat cttacggatg 540

gcatgacagt aagagaatta tgcagtgctg ccataaccat gagtgataac actgcggcca 600

acttacttct gacaacgatc ggaggaccga aggagctaac cgcttttttg cacaacatgg 660

gggatcatgt aactcgcctt gatcgttggg aaccggagct gaatgaagcc ataccaaacg 720

acgagcgtga caccacgatg cctgtagcaa tggcaacaac gttgcgcaaa ctattaactg 780

gcgaactact tactctagct tcccggcaac aattaataga ctggatggag gcggataaag 840

ttgcaggacc acttctgcgc tcggcccttc cggctggctg gtttattgct gataaatctg 900

gagccggtga gcgtgggtct cgcggtatca ttgcagcact ggggccagat ggtaagccct 960

cccgtatcgt agttatctac acgacgggga gtcaggcaac tatggatgaa cgaaatagac 1020

agatcgctga gataggtgcc tcactgatta agcattggta actgtcagac caagtttact 1080

catatatact ttagattgat ttaaaacttc atttttaatt taaaaggatc taggtgaaga 1140

tcctttttga taatctcatg accaaaatcc cttaacgtga gttttcgttc cactgagcgt 1200

cagaccccgt agaaaagatc aaaggatctt cttgagatcc tttttttctg cgcgtaatct 1260

gctgcttgca aacaaaaaaa ccaccgctac cagcggtggt ttgtttgccg gatcaagagc 1320

taccaactct ttttccgaag gtaactggct tcagcagagc gcagatacca aatactgttc 1380

ttctagtgta gccgtagtta ggccaccact tcaagaactc tgtagcaccg cctacatacc 1440

tcgctctgct aatcctgtta ccagtggctg ctgccagtgg cgataagtcg tgtcttaccg 1500

ggttggactc aagacgatag ttaccggata aggcgcagcg gtcgggctga acggggggtt 1560

cgtgcacaca gcccagcttg gagcgaacga cctacaccga actgagatac ctacagcgtg 1620

agctatgaga aagcgccacg cttcccgaag ggagaaaggc ggacaggtat ccggtaagcg 1680

gcagggtcgg aacaggagag cgcacgaggg agcttccagg gggaaacgcc tggtatcttt 1740

atagtcctgt cgggtttcgc cacctctgac ttgagcgtcg atttttgtga tgctcgtcag 1800

gggggcggag cctatggaaa aacgccagca acgcggcctt tttacggttc ctggcctttt 1860

gctggccttt tgctcacatg ttctttcctg cgttatcccc tgattctgtg gataaccgta 1920

ttaccgcctt tgagtgagct gataccgctc gccgcagccg aacgaccgag cgcagcgagt 1980

cagtgagcga ggaagcggaa gagcgcccaa tacgcaaacc gcctctcccc gcgcgttggc 2040

cgattcatta atgcagctgg cacgacaggt ttcccgactg gaaagcgggc agtgagcgca 2100

acgcaattaa tgtgagttag ctcactcatt aggcacccca ggctttacac tttatgcttc 2160

cggctcgtat gttgtgtgga attgtgagcg gataacaatt tcacacagga aacagctatg 2220

accatgatta cgccaagctt gcatgcaggc ctctgcagtc gacgggcccg tctccgggcc 2280

ccccgctagt acggccgcta ctattaagat cctcctcgga tattaacttc tgctcaccgt 2340

tgaggtcttc ctcactgatc aatttctgtt ctccattcaa gtcctcttca gaaatgagct 2400

tttgctcaga acggccgcac agctctaatt cgcggccgcc tgagagtgca ccataccacc 2460

ttttcaattc atcatttttt ttttattctt ttttttgatt tcggtttcct tgaaattttt 2520

ttgattcggt aatctccgaa cagaaggaag aacgaaggaa ggagcacaga cttagattgg 2580

tatatatacg catatgtagt gttgaagaaa catgaaattg cccagtattc ttaacccaac 2640

tgcacagaac aaaaacctgc aggaaacgaa gataaatcat gtcgaaagct acatataagg 2700

aacgtgctgc tactcatcct agtcctgttg ctgccaagct atttaatatc atgcacgaaa 2760

agcaaacaaa cttgtgtgct tcattggatg ttcgtaccac caaggaatta ctggagttag 2820

ttgaagcatt aggtcccaaa atttgtttac taaaaacaca tgtggatatc ttgactgatt 2880

tttccatgga gggcacagtt aagccgctaa aggcattatc cgccaagtac aattttttac 2940

tcttcgaaga cagaaaattt gctgacattg gtaatacagt caaattgcag tactctgcgg 3000

gtgtatacag aatagcagaa tgggcagaca ttacgaatgc acacggtgtg gtgggcccag 3060

gtattgttag cggtttgaag caggcggcag aagaagtaac aaaggaacct agaggccttt 3120

tgatgttagc agaattgtca tgcaagggct ccctatctac tggagaatat actaagggta 3180

ctgttgacat tgcgaagagc gacaaagatt ttgttatcgg ctttattgct caaagagaca 3240

tgggtggaag agatgaaggt tacgattggt tgattatgac acccggtgtg ggtttagatg 3300

acaagggaga cgcattgggt caacagtata gaaccgtgga tgatgtggtc tctacaggat 3360

ctgacattat tattgttgga agaggactat ttgcaaaggg aagggatgct aaggtagagg 3420

gtgaacgtta cagaaaagca ggctgggaag catatttgag aagatgcggc cagcaaaact 3480

aaaaaactgt attataagta aatgcatgta tactaaactc acaaattaga gcttcaattt 3540

aattatatca gttattaccc tatgcggtgt gaaataccgc acagatgcgt aaggagaaaa 3600

taccgcatca ggaaattgta gcggccgcgg ggaaatccgc tagtacggcc gctactatta 3660

agatcctcct cggatattaa cttctgctca ccgttgaggt cttcctcact gatcaatttc 3720

tgttctccat tcaagtcctc ttcagaaatg agcttttgct cagaacggcc gcacagctcc 3780

aggtcgggat cggaacaaaa gctggagctc agtttatcat tatcaatact cgccatttca 3840

aagaatacgt aaataattaa tagtagtgat tttcctaact ttatttagtc aaaaaattag 3900

ccttttaatt ctgctgtaac ccgtacatgc ccaaaatagg gggcgggtta cacagaatat 3960

ataacatcgt aggtgtctgg gtgaacagtt tattcctggc atccactaaa tataatggag 4020

cccgcttttt aagctggcat ccagaaaaaa aaagaatccc agcaccaaaa tattgttttc 4080

ttcaccaacc atcagttcat aggtccattc tcttagcgca actacagaga acaggggcac 4140

aaacaggcaa aaaacgggca caacctcaat ggagtgatgc aacctgcctg gagtaaatga 4200

tgacacaagg caattgaccc acgcatgtat ctatctcatt ttcttacacc ttctattacc 4260

ttctgctctc tctgatttgg aaaaagctga aaaaaaaggt tgaaaccagt tccctgaaat 4320

tattccccta cttgactaat aagtatataa agacggtagg tattgattgt aattctgtaa 4380

atctatttct taaacttctt aaattctact tttatagtta gtcttttttt tagttttaaa 4440

acaccagaac ttagtttcga cggattctag aactagtgga tcccccgggc tgcaggaatt 4500

cgatatcaag cttatcgata ccgtcgacct cgagtcatgt aattagttat gtcacgctta 4560

cattcacgcc ctccccccac atccgctcta accgaaaagg aaggagttag acaacctgaa 4620

gtctaggtcc ctatttattt ttttatagtt atgttagtat taagaacgtt atttatattt 4680

caaatttttc ttttttttct gtacagacgc gtgtacgcat gtaacattat actgaaaacc 4740

ttgcttgaga aggttttggg acgctcgaag gctttaattt gcggccggta aattcactgg 4800

ccgtcgtttt acaacgtcgt gactgggaaa accctggcgt tacccaactt aatcgccttg 4860

cagcacatcc ccctttcgcc agctggcgta atagcgaaga ggcccgcacc gatcgccctt 4920

cccaacagtt gcgcagcctg aatggcgaat ggcgcctgat gcggtatttt ctccttacgc 4980

atctgtgcgg tatttcacac cgcatatggt gcactctcag tacaatctgc tctgatgccg 5040

catagttaag ccagccccga cacccgccaa cacccgctga cgcgccctga cgggcttgtc 5100

tgctcccggc atccgcttac agacaagctg tgaccgtctc cgggagctgc atgtgtcaga 5160

ggttttcacc gtcatcaccg aaacgcgcga 5190

<210>    31
<211>    1365
<212>    DNA
<213>    Unknown

<220>
<223>    Gdh1


<400>    31
atgtcagagc cagaatttca acaagcttac gaagaagttg tctcctcttt ggaagactct      60

actcttttcg aacaacaccc agaatacaga aaggttttgc caattgtttc tgttccagaa     120

agaatcatac aattcagagt cacctgggaa aatgacaagg gtgaacaaga agttgctcaa     180

ggttacagag tgcaatataa ctccgccaag ggtccataca agggtggtct acgtttccat     240

ccttccgtga acttgtctat cttgaaattc ttgggtttcg aacaaatctt caagaactcc     300

ttgaccggcc tagacatggg tggtggtaaa ggtggtctat gtgtggactt gaagggaaga     360

tctaataacg aaatcagaag aatctgttat gctttcatga gagaattgag cagacacatt     420

ggtcaagaca ctgacgtgcc agctggtgat atcggtgttg gtggtcgtga aattggttac     480

ctgttcggtg cttacagatc atacaagaac tcctgggaag gtgtcttaac cggtaagggt     540

ttgaactggg gtggttcttt gatcagacca gaagccactg gttacggttt agtttactat     600

actcaagcta tgatcgacta tgccacaaac ggtaaggaat ctttcgaagg taagcgcgtc     660

accatctctg gtagtggtaa cgttgctcaa tacgctgcct tgaaggttat tgagctaggt     720

ggtactgtcg tttccctatc tgactccaag ggttgtatca tctctgaaac tggtatcacc     780

tccgaacaag tcgctgatat ttccagtgct aaggtcaact tcaagtcctt ggaacaaatc     840

gtcaacgaat actctacttt ctccgaaaac aaagtgcaat acattgctgg tgctcgtcca     900

tggacccacg tccaaaaggt cgacattgct ttgccatgtg ccacccaaaa tgaagtcagc     960

ggtgaagaag ccaaggcctt ggttgctcaa ggtgtcaagt ttattgccga aggttccaac    1020

atgggttcca ctccagaagc tattgccgtc tttgaaactg ctcgttccac cgccactgga    1080

ccaagcgaag ctgtttggta cggtccacca aaggctgcta acttgggtgg tgttgctgtt    1140

tctggtttag aaatggcaca aaactctcaa agaatcacat ggactagcga aagagttgac    1200

caagagttga agagaattat gatcaactgt ttcaatgaat gtatcgacta tgccaagaag    1260

tacactaagg acggtaaggt cttgccatct ttggtcaaag gtgctaatat cgcaagtttc    1320

atcaaggtct ctgatgctat gtttgaccaa ggtgatgtat tttaa                    1365


<210>    32
<211>    1470
<212>    DNA
<213>    Unknown

<220>

<223>    Gnd1

<400>    32
atgtctgctg atttcggttt gattggtttg gccgtcatgg gtcaaaattt gatcttgaac      60

gctgctgacc acggtttcac tgtttgtgct tacaacagaa ctcaatccaa ggtcgaccat     120

ttcttggcca atgaagctaa gggcaaatct atcatcggtg ctacttccat tgaagatttc     180

atctccaaat tgaagagacc tagaaaggtc atgcttttgg ttaaagctgg tgctccagtt     240

gacgctttga tcaaccaaat cgtcccactt ttggaaaagg gtgatattat catcgatggt     300

ggtaactctc acttcccaga ttctaataga cgttacgaag aattgaagaa gaagggtatt     360

cttttcgttg gttctggtgt ctccggtggt gaggaaggtg cccgttacgg tccatctttg     420

atgccaggtg gttctgaaga agcttggcca catattaaga acatcttcca atccatctct     480

gctaaatccg acggtgaacc atgttgcgaa tgggttggcc cagccggtgc tggtcactac     540

gtcaagatgg ttcacaacgg tattgaatac ggtgatatgc aattgatttg tgaagcttat     600

gacatcatga agagattggg tgggtttacc gataaggaaa tcagtgacgt ttttgccaaa     660

tggaacaatg gtgtcttgga ttccttcttg gtcgaaatta ccagagatat tttgaaattc     720

gacgacgtcg acggtaagcc attagttgaa aaaatcatgg atactgctgg tcaaaagggt     780

actggtaagt ggactgccat caacgccttg gatttgggta tgccagttac tttgattggt     840

gaagctgtct ttgcccgttg tctatctgct ttgaagaacg agagaattag agcctccaag     900

gtcttaccag gcccagaagt tccaaaagac gccgtcaagg acagagaaca atttgtcgat     960

gatttggaac aagctttgta tgcttccaag attatttctt acgctcaagg tttcatgttg    1020

atccgtgaag ctgctgctac ttatggctgg aaactaaaca accctgccat cgctttgatg    1080

tggagaggtg gttgtatcat tagatctgtt ttcttgggtc aaatcacaaa ggcctacaga    1140

gaagaaccag atttggaaaa cttgttgttc aacaagttct tcgctgatgc cgtcaccaag    1200

gctcaatctg gttggagaaa gtcaattgcg ttggctacca cctacggtat cccaacacca    1260

gccttttcca ccgctttgtc tttctacgat gggtacagat ctgaaagatt gccagccaac    1320

ttactacaag ctcaacgtga ctactttggt gctcacactt tcagagtgtt gccagaatgt    1380

gcttctgaca acttgccagt agacaaggat atccatatca actggactgg ccacggtggt    1440

aatgtttctt cctctacata ccaagcttaa                                     1470

<210>    33
<211>    3211
<212>    DNA
<213>    Unknown

<220>
<223>    Gdh2

<400>    33
aatatcatcc atgtcggact atcacgtgtt tgattttccc ggtaaggacc tgcagagaga      60

ggaagtgata gatttgctag atcagcaagg gtttattccc gacgatttga tcgaacaaga     120

agtagattgg ttttataact cattgggtat tgacgatttg ttcttctcga gagaatctcc     180

ccaattaatc tcgaatatca tacattcttt gtatgcttca aagctagatt tctttgcgaa     240

gtccaaattc aacggaattc agccaaggct attcagcatt aaaaacaaaa ttataactaa     300

tgataatcat gccatcttta tggaatctaa tactggtgtc agcataagcg attctcagca     360

aaaaaacttt aaatttgcta gtgacgccgt cggaaacgat actttggagc atggtaagga     420

taccatcaaa aaaaatagga ttgaaatgga tgattcttgt ccaccttatg aattagattc     480

cgaaattgat gaccttttcc tggataacaa gtctcaaaaa aactgcagat tagtttcttt     540

ttgggctcca gaaagcgaat taaagctaac ttttgtttat gagagtgttt accctaatga     600

tgatccagcc ggcgtagata tttcctctca ggatttgctg aaaggtgata ttgaatcgat     660

tagtgataag accatgtaca aagtttcgtc gaacgaaaat aaaaaactat acggtctctt     720

acttaagttg gttaaagaaa gagaaggtcc tgtcattaag actactcgct ccgtagaaaa     780

taaggatgaa attaggttat tagtcgctta caagcgattc accactaagc gttattactc     840

tgctttgaac tctttgttcc actattacaa gttgaaacct tctaagttct atttagagtc     900

gtttaatgtt aaggatgatg acatcattat cttttccgtt tatttgaacg agaaccagca     960

attggaagat gttctacttc acgatgtgga ggcagcattg aaacaggttg aaagagaagc    1020

ttcattgcta tacgctatcc caaacaattc tttccatgag gtttaccaga gacgtcaatt    1080

ctcgcccaaa gaagctatat atgctcatat tggtgctata ttcattaacc attttgttaa    1140

tcgtttaggc tctgattatc aaaacctttt atctcaaatc accattaagc gtaatgatac    1200

tactcttttg gagattgtag aaaacctaaa aagaaagtta agaaatgaaa ccttaactca    1260

gcaaactatt atcaacatca tgtcgaagca ttacactata atttccaagt tgtataaaaa    1320

ttttgctcaa attcactatt atcataatag tactaaagat atggagaaga cattatcttt    1380

tcaaagactg gaaaaagtgg agccttttaa gaatgaccaa gagttcgaag cttacttgaa    1440

taaattcatt ccaaatgatt cacctgattt gttgatcctg aaaacactga acatcttcaa    1500

caagtctatt ttgaagacaa atttctttat tacaagaaaa gtagcaatat cattcagatt    1560

agatccttcc ctggtgatga caaaattcga atatccagag acaccctatg gtatattttt    1620

tgtcgttggt aatactttca aagggttcca tatcaggttc agagatatcg caaggggcgg    1680

tattcgtata gtctgttcca ggaatcagga tatttatgat ttgaattcca agaacgttat    1740

tgatgagaac tatcaattgg cctctactca gcaacgtaaa aataaggata ttccagaggg    1800

tggctctaaa ggtgtcatct tattgaaccc aggattggta gaacatgacc agacatttgt    1860

cgccttttcc caatatgtgg atgcaatgat tgacattcta atcaacgatc cattaaagga 1920

aaactatgtc aaccttttac caaaggagga aatattattt tttggcccag atgaaggaac 1980

tgctggtttc gtggattggg caactaacca tgctcgtgtg aggaactgcc catggtggaa 2040

atcatttttg actggaaaat ccccatcttt gggtggtatt ccccatgacg aatatggtat 2100

gacttctctg ggtgttcgtg cttatgttaa taaaatttac gaaactttaa acttgacaaa 2160

ttctactgtt tacaaattcc aaactggtgg tccggatggt gatttgggat ccaatgaaat 2220

tcttttatct tcgccaaacg aatgttattt ggcaattctg gacggttcag gtgtcctgtg 2280

tgatcctaaa ggtttagata aagatgaatt atgccgcttg gcacatgaaa ggaaaatgat 2340

ttccgatttc gacacttcca aattatcaaa caacggattt tttgtttctg tggatgcaat 2400

ggatatcatg ctaccaaatg gtacaattgt agctaacggc acaaccttca gaaacacctt 2460

tcatactcaa attttcaaat ttgtggatca tgtcgacatt tttgttccat gcggtggtag 2520

accaaactca attactctaa ataatctaca ttattttgtt gacgaaaaga ctgggaaatg 2580

taaaattcca tatattgtgg agggtgccaa tctatttata acgcaacctg ctaaaaatgc 2640

tttggaggaa catggctgta ttctgttcaa agatgcttct gcaaacaaag gtggtgtcac 2700

atcttcatca atggaagtgt tggcctcact agcgcttaac gataacgact tcgtgcacaa 2760

atttattgga gatgttagtg gtgagaggtc tgcgttgtac aagtcgtacg ttgtagaagt 2820

gcagtcaaga attcagaaaa atgctgaatt agagtttggt cagttatgga atttgaatca 2880

actaaatgga acccacattt cagaaatttc aaaccaattg tccttcacta taaacaaatt 2940

gaacgacgat ctagttgctt ctcaagagtt gtggctcaat gatctaaaat taagaaacta 3000

cctattgttg gataaaataa ttccaaaaat tctgattgat gttgctgggc ctcagtccgt 3060

attggaaaac attccagaga gctatttgaa agttcttctg tcgagttact tatcaagcac 3120

ttttgtttac cagaacggta tcgatgttaa cattggaaaa ttcttggaat ttattggtgg 3180

gttaaaaaga gaagcggagg caagtgcttg a 3211

<210> 34
<211> 2232
<212> DNA
<213> Unknown

<220>
<223> Stb5

<400> 34
atggatggtc ccaattttgc acatcaaggc gggagatcac aacgtactac tgaattgtat 60

tcgtgcgcac gatgcagaaa attaaagaag aagtgtggta aacaaatacc gacatgtgca 120

aactgcgata aaaatggggc acactgttca tatccaggta gagccccaag acgtaccaag 180

aaggagttag cggatgctat gctacgaggg gaatatgttc cagtgaaaag gaacaagaag 240

gtaggaaaaa gcccattgag cactaagagc atgccaaact cttctagtcc gctatccgca 300

aatggcgcta taactcccgg gttttcgcct tacgaaaacg atgatgcaca taagatgaaa 360

cagctaaaac cgtcagatcc aataaatctt gtcatggggg caagtccaaa ttctagcgaa 420

ggtgtctcat cgctaatttc ggtgctaaca tcgctgaatg ataattctaa tccttcttcg 480

cacttatcct ctaatgaaaa ttccatgatt ccttcccgat cattgccagc ttccgtgcag 540

caaagttcga caacttcatc attcggagga tataacacgc cttcaccact aattagcagt 600

catgtgcctg cgaacgccca agccgtaccg ctacaaaaca acaatcgcaa tactagcaac 660

ggggataacg gcagtaatgt taaccacgat aataacaatg gcagtaccaa cacaccgcaa 720

ttgagtctta ccccatatgc aaacaattca gcccccaatg ggaaattcga ttctgtgccg 780

gttgatgcat cctcgatcga atttgaaact atgtcctgtt gctttaaagg tggtagaaca 840

acatcgtggg tcagagagga tggctcgttc aagtcaattg atagatcctt actggacagg 900

ttcattgccg catacttcaa acacaatcac cgtctatttc ccatgattga taaaatagca 960

ttcctaaatg acgccgcgac aattactgat ttcgaaaggt tatatgacaa caaaaactac 1020

cctgacagct ttgttttcaa agtatacatg atcatggcta ttggttgtac aactttacag 1080

cgtgctggta tggtttctca ggacgaagag tgtctgagtg aacatttggc ttttttggcc 1140

atgaaaaaat ttcgtagtgt tataatttta caagatatcg aaactgtacg atgcctattg 1200

ttgttgggta tttattcgtt ttttgagcca aagggctcct cgtcatggac aattagtggt 1260

atcatcatgc gattgaccat aggattaggt ttaaatagag agttaactgc caaaaaactc 1320

aagagcatgt ctgctttaga agcagaggca agatatagag tgttttggag tgcttactgc 1380

tttgaaaggc tagtatgcac ctcgttgggc cgtatatccg ggatcgacga cgaagacatc 1440

actgtgccac taccgagggc gttgtatgtg gatgaaagag acgatttaga gatgaccaag 1500

ttgatgatat cattaaggaa aatgggcggt cgcatttata aacaagtcca ctctgtaagt 1560

gcagggcgac aaaagttaac catcgaacaa aagcaggaaa tcataagtgg attacgcaag 1620

gagctagacg aaatttattc tcgagaatca gaaagaagga aactgaaaaa atctcaaatg 1680

gatcaggtgg agagggagaa caattctact acaaatgtaa tatccttcca tagttctgag 1740

atttggctag caatgagata ctcccagttg caaatcttac tatacagacc atctgcattg 1800

atgccaaaac cgcccattga ctcactatcc actctaggag agttttgctt gcaagcctgg 1860

aaacatactt atacactgta caagaagcgg ttattaccct tgaactggat aacccttttc 1920

agaacattaa ccatttgtaa cactatctta tactgtcttt gccagtggag catcgacctc 1980

attgaaagta aaatcgaaat tcaacagtgt gtggaaatac taagacattt cggtgaaaga 2040

tggatttttg ctatgagatg cgcggatgtt ttccaaaaca ttagcaacac aattctcgat 2100

attagtttaa gccatggtaa agttcctaat atggaccaat taacaagaga gttatttggc 2160

gccagcgatt catatcaaga catattagac gaaaacaatg ttgacgtctc ttgggttgat 2220

aaacttgtat ga 2232

## Description

### Technical Field

The present invention relates to yeast for increasing ginsenoside production, and a method for producing ginsenosides using yeast.

### Background Art

Saponins are glycosides widely present in plant systems, in which the non-sugar moiety consists of various cyclic compounds. Triterpene saponins, which are saponin components contained as major physiologically active components in ginseng or red ginseng, have chemical structures different from those of saponins discovered in other plants, and thus these ginseng saponins are called ginsenosides, having the meaning of ginseng glycosides, so as to distinguish them from saponins in other plants. Ginsenosides can be classified into three types according to the structure of ginsenosides: protopanaxadiol-type (PPD-type) ginsenosides, protopanaxatriol-type (PPT-type) ginsenosides, and oleanolic acid-type ginsenosides.

Pharmacological study results of ginseng have increased public interest in ginseng saponins (*i.e.,* ginsenosides), and thus there is a growing need for the mass production of ginsenosides. However, the mass production of useful materials of ginseng by conventional cultivation methods has problems in that it would require a long culture period of 4 to 6 years, presenting difficulties in controlling pests and diseases by shading culture, rotation culture, *etc.,* and thus there is an urgent need for the development of a new alternative production method.

Recently, many ginseng saponin-related genes have been discovered based on bioengineering technology, and as a result, the development of techniques for mass production of ginsenosides in yeast using these genes has recently started to receive attention as well. Since ginsenosides are biosynthesized in plants through the isoprenoid biosynthetic pathways including the mevalonic acid biosynthetic pathway, synthetic biology studies have been attempted to develop ginsenoside-producing strains by redesigning the ergosterol biosynthetic pathway of yeast.

Recently, China's Huang and Zhang joint research team reported that it had succeeded in producing protopanaxadiol by expressing, in *Saccharomyces cerevisiae* (yeast), the protopanaxadiol dammarenediol-II synthase gene and the protopanaxadiol synthase gene of ginseng along with the NADPH-cytochrome P450 reductase gene obtained from *Arabidopsis thaliana* (Dai, Z. et al., (2013) Metabolic engineering of Saccharomyces cerevisiae for production of ginsenosides. Metab. Eng. 20: 146 to 156.).

In the future, it is expected that ginsenoside-producing synthetic yeast will be able to provide, through more optimized work, a cost-effective production process that can replace the complex process of extracting ginsenosides from plants.

Under the circumstances, the present inventors have made efforts to increase the amount of ginsenoside production using yeast. As a result, they have developed ginsenoside-producing yeast, where the expression levels of NADPH biosynthesis-related genes are altered, and a method for preparing the yeast, and they have confirmed that the yeast produces an increased amount of protopanaxadiol (*i.e.,* an intermediate product of ginsenoside biosynthesis) compared to the existing yeast having the ginsenoside-producing ability, thereby completing the present invention.

### Technical Problem

An object of the present invention is to provide yeast for producing ginsenosides.

Another object of the present invention is to provide a method for preparing the yeast.

Still another object of the present invention is to provide a method for producing ginsenosides in high yield using the yeast.

### Technical Solution

The preferred embodiments are described in detail is as follows. Meanwhile, respective descriptions and embodiments disclosed in the present invention may also be applied to other descriptions and embodiments. That is, all combinations of various elements disclosed in the present invention fall within the scope of the present invention. Further, the scope of the present invention is not limited by the specific description below.

In order to achieve the above objects, an aspect of the present invention provides yeast for producing ginsenosides, in which the expression levels of NADPH biosynthesis-related genes are changed compared to their endogenous expression levels.

In order to achieve the above objects, another aspect of the present invention provides a composition for producing ginsenosides, containing yeast for producing ginsenosides in which the expression levels of NADPH biosynthesis-related genes are changed compared to their endogenous expression levels.

As used herein, the term "reduced nicotinamide adenine dinucleotide phosphate (NADPH)" refers to a kind of coenzyme which participates, as an electron donor, in many reactions of oxidoreductase and dehydrogenase along with NADH, which shares the structure of nicotinamide adenine dinucleotide, thereby providing reducing power. The oxides of these coenzymes (*i.e.,* NAD⁺ and NADP⁺) are known to have an important role of receiving the energy generated in the catabolic reaction in the form of electrons and protons, and participate in the oxidation-reduction enzyme reaction as an electron receptor.

The present invention is characterized in that the ginsenoside production is increased by changing the expression levels of the NADPH biosynthesis-related genes (increased or decreased). For the purposes of the present invention, any gene among the NADPH biosynthesis-related genes involved in the mass production of ginsenosides, specifically for the production of protopanaxadiol (PPD), can be used without limitation.

In embodiments of the present invention, strains where the genes related to the NADPH biosynthetic pathway are modified were prepared, and thereby the amounts of NADPH production for wild-type yeast cells and transformed yeast cells were confirmed (Examples 1 to 3). Specifically, various kinds of strains (*e.g.,* strains where *Ald2* gene or *Gdh1* gene is inactivated; strains where *Gnd1* gene, *Gdh2* gene, *Ald6* gene, *Zwf1* gene, or *Stb5* gene is overexpressed; strains where *Ald2* gene is inactivated and *Ald6* gene is overexpressed; strains where *Gdh1* gene is inactivated and *Gdh2* gene is overexpressed; *etc.)* were prepared so as to examine whether there is any increase in the amount of NADPH production in these strains.

The NADPH biosynthesis-related gene may be at least one selected from the group consisting of *Ald2, Gdh1, Gnd1, Gdh2, Ald6, Stb5, Zwf1,* and *Zms1* genes, more specifically at least one selected from the group consisting of *Ald2, Ald6, Zwf1,* and *Zms1* genes, but the NADPH biosynthesis-related genes are not limited thereto. The NADPH biosynthesis-related gene may be 9 genes, 8 genes, 7 genes, 6 genes, 5 genes, 4 genes, 3 genes, 2 genes, or 1 gene.

The sequences of the genes or enzymes encoded by these genes may be obtained from a known database (*e.g.,* NCBI, *etc.),* but the available sources are not limited thereto.

ALD2, which is the enzyme encoded by the *Ald2* gene, is an enzyme converting acetaldehyde to acetate, and it produces NADH during the process. Additionally, ALD6 is an enzyme that performs the same reaction as ALD2 (*i.e*. isozyme), and likewise produces NADPH during the process. Additionally, ZMS1, which is an enzyme encoded by the *Zms1* gene, is a transcription regulation factor and is known to induce overexpression of the *Ald6* gene.

Specifically, the *Ald2* may have a gene sequence of SEQ ID NO: 1 and the *Ald6* may have a gene sequence of SEQ ID NO: 2, but any sequence encoding ALD2 and ALD6 may be included without limitation.

ZWF1, which is the enzyme encoded by the *Zwfl* gene, is an essential enzyme in the pentose phosphate pathway (PP pathway), and it catalyzes the first step of the PP pathway. ZWF1 is known to be involved in oxidative stress, and to be responsible for production of pentose and NADPH in the cell.

Specifically, the *Zwfl* may have a gene sequence of SEQ ID NO: 3 and *Zms1* may have a gene sequence of SEQ ID NO: 4, but any sequence encoding ZWF1 and ZMS1 may be included without limitation. In addition, *Gdh1, Gnd1, Gdh2,* and *Stb5* may have each of the gene sequences of SEQ ID NO: 31, SEQ ID NO: 32, SEQ ID NO: 33, and SEQ ID NO: 34, but any sequence encoding GDH1, GND1, GDH2, and STB5 enzymes may be included without limitation.

For the purposes of the present invention, any gene which is involved in the increase of NADPH biosynthesis, thus being capable of increasing protopanaxadiol (PPD)-based ginsenosides, may be applicable without limitation.

Additionally, in the present invention, the above genes can include not only those sequences described above, but also those genes which have a homology to the above sequences of at least 80%, specifically at least 90%, more specifically at least 95%, and even more specifically at least 99%. Additionally, it is apparent that any gene sequence which has a nucleotide sequence having a homology to the above sequences, and encodes an enzyme which exhibits effects substantially the same as or corresponding to those of the above enzymes may be included without limitation. Additionally, it is apparent that any nucleotide sequence having such a homology falls within the scope of the present invention, even if the nucleotide sequence may include deletion, modification, substitution, or addition in part of the sequence.

In the above, the term "homology" refers to the degree of similarity to a given nucleotide sequence, and it may be expressed as a percentage (%). In the present specification, a homologous sequence having an activity the same as or similar to that of a given nucleotide sequence may be expressed as "% homology". For example, the homology may be identified using standard software, specifically BLAST 2.0, for calculating parameters (*e.g*., score, identity, similarity, *etc*.) or by comparing sequences through hybridization experiments under defined stringent conditions. Appropriate hybridization conditions may be determined within the scope of the art and by methods well known to those skilled in the art (*e.g.,* J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

The yeast of the present invention is characterized in that the expression levels of the NADPH biosynthesis-related proteins are changed compared to their endogenous levels.

As used herein, the term "endogenous expression level" refers to the expression level of mRNA or protein of a microorganism that is expressed in a natural state or in its parent strain before the expression level of the corresponding protein undergoes modification. This is essentially the degree to which a given mRNA or protein is produced in a cell or tissue of a strain, under normal circumstances or prior to controlling the expression of a specific protein. The endogenous expression levels can be compared between strain types, cell types, and tissues, or may be compared to expression levels induced by some stimuli. Specifically, the endogenous expression level may be an expression level of mRNA or a protein expressed in a microorganism where the expression of NADPH biosynthesis-related proteins are not regulated.

The above term "change" may be used to include the increase or decrease or both. For the purposes of the present invention, the term "change" may include decreasing the expression of a particular gene by inactivating the gene and increasing the expression of a particular gene by overexpressing the gene. That is, "decrease" may be used to refer to "decrease in expression level compared to its endogenous expression level" and "increase" may be used to refer to "increase in expression level compared to its endogenous expression level".

Specifically, the phrase "increase in expression level compared to its endogenous expression level" means that the gene encoding the corresponding polypeptide is expressed at a higher level compared to when the gene is in a natural state or before modification, and thus the corresponding polypeptide with functional ability is produced in a large amount.

Specifically, the increase in expression level compared to its endogenous expression level may be performed by the following methods:
1) a method for increasing the copy number of the polynucleotide that encodes the protein,
2) a method for modifying the expression control sequence so as to increase the expression of the polynucleotide,
3) a method for modifying the polynucleotide sequence on the chromosome so as to enhance the activity of the protein, or
4) a method for modification so as to enhance the activity of the protein by a combination of the above methods 1) to 3), *etc.,* but the methods are not limited thereto.

The method of increasing the copy number of the polynucleotide in method 1) may be performed in the form where the polynucleotide is operably linked to a vector, or by inserting the polynucleotide into the chromosome in a host cell, but the method is not particularly limited thereto. Specifically, it may be performed by inserting a vector into a host cell, in which the vector is operably linked to a polynucleotide encoding the protein of the present invention, and can replicate and function regardless of the host cell. Alternatively, it may be performed by a method for increasing the copy number of the polynucleotide in the chromosome in the host cell by inserting a vector into the host cell, in which the vector is operably linked to the polynucleotide and is able to insert the polynucleotide into the chromosome in the host cell. The introduction may be performed by those skilled in the art by appropriately selecting a known transformation method, and the enzyme may be produced by the expression of the introduced polynucleotide in a host cell, and thereby its activity can be increased.

Next, the method 2) of modifying the expression control sequence so as to increase the expression of the polynucleotide may be performed by inducing a mutation on the sequence by deletion, insertion, or non-conservative or conservative substitution of a nucleic acid sequence, or a combination thereof, such that the activity of the expression control sequence can be further enhanced, or by replacing the sequence with a nucleic acid sequence having enhanced activity, but the method is not particularly limited thereto. The expression control sequence may include a promoter, an operator sequence, a sequence encoding a ribosomal binding site, a sequence controlling the termination of transcription and translation, *etc.,* but the method is not particularly limited thereto.

In addition, the method 3) for modifying the polynucleotide sequence on the chromosome may be performed by inducing a mutation on the sequence by deletion, insertion, or non-conservative or conservative substitution of a nucleic acid sequence, or a combination thereof, such that the activity of the polynucleotide sequence can be further enhanced, or by replacing the sequence with a polynucleotide sequence having enhanced activity, but the method is not particularly limited thereto.

Finally, the method 4) of modifying so as to enhance the activity of the protein by a combination of the above methods 1) to 3) may be performed by applying at least one method among a method of increasing the copy number of the polynucleotide that encodes the protein; a method of modifying the expression control sequence so as to increase the expression of the polynucleotide; a method for modifying the polynucleotide sequence on the chromosome; and a method for modifying an exogenous polynucleotide exhibiting the activity of the enzyme, or modifying a codon-optimized modified polynucleotide thereof.

As used herein, the term "vector" refers to a DNA construct including the nucleotide sequence of the polynucleotide encoding a target protein, in which the target protein is operably linked to a suitable control sequence so that the target protein can be expressed in an appropriate host. The control sequence may include a promoter capable of initiating transcription, any operator sequence capable of controlling such transcription, a sequence encoding an appropriate mRNA ribosome-binding domain, and a sequence for controlling the termination of transcription and translation, but the control sequence is not limited thereto. The vector, after being transformed into a suitable host cell, may be replicated or function irrespective of the host genome, or may be integrated to the genome itself.

The vector used in the present invention may not be particularly limited as long as the vector is replicable in the host cell, and any vector known in the art may be used. Examples of the vector conventionally used may include natural or recombinant plasmids, and the replication origin may include the autonomous replication sequence (ARS) of yeast. The autonomous replication sequence may be stabilized by the centrometric sequence (CEN) of yeast. The promoter may be one selected from the group consisting of CYC promoter, TEF promoter, GPD promoter, PGK promoter, ADH promoter, *etc.* The terminator may be selected from the group consisting of PGK1, CYC1, GAL1, *etc.* The vector may further include a selection marker.

The vector used in the present invention is not particularly limited, but any known expression vector may be used. Additionally, a polynucleotide encoding a target protein may be inserted into the chromosome in a cell using a vector for chromosomal insertion. The insertion of the polynucleotide into the chromosome may be performed using any method known in the art, *e.g.,* by homologous recombination, but the insertion is not limited thereto. A selection marker for confirming the insertion of the vector into the chromosome may be further included. The selection marker is used for selection of cells transformed with the vector, *i.e.,* in order to confirm whether the target nucleic acid molecule has been inserted, and markers capable of providing selectable phenotypes such as drug resistance, auxotrophy, resistance to cytotoxic agents, and expression of surface proteins may be used. Under the circumstances where selective agents are treated, only the cells capable of expressing the selection markers can survive or express other phenotypic traits, and thus the transformed cells can easily be selected.

As used herein, the term "transformation" refers to a process of introducing a vector, which includes a target protein-encoding polynucleotide, into a host cell such that the protein encoded by the polynucleotide can be expressed in the host cell. It does not matter whether the transformed polynucleotide is inserted into the chromosome of the host cell and located thereon or located outside of the chromosome, as long as the transformed polynucleotide can be expressed in the host cell. Additionally, the polynucleotide may include DNA and RNA encoding the target protein. The polynucleotide may be introduced in any form, as long as the polynucleotide can be introduced into the host cell and expressed therein. For example, the polynucleotide may be introduced into the host cell in the form of an expression cassette, which is a gene construct including all elements required for its autonomous expression. The expression cassette may include a promoter, a transcription termination signal, a ribosome binding site, and a translation termination signal that are operably linked to the polynucleotide. The expression cassette may be in a form of an expression vector capable of self-replication. Further, the polynucleotide may be introduced into the host cell as is to be operably linked to the sequence required for its expression in the host cell, but the form of the expression cassette is not limited thereto.

The transformation method may include any method which can introduce nucleic acids into a cell, and the transformation may be performed by selecting an appropriate technique as known in the art according to the host cell. For example, the method may include electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate/DMSO method, *etc.,* but the method is not limited thereto.

Additionally, as used herein, the term "operably linked" refers to a functional linkage between a promoter sequence, which initiates and mediates the transcription of the polynucleotide encoding the target protein of the present invention, and the polynucleotide sequence. The operable linkage may be prepared by genetic recombination technology known in the art, and site-specific DNA cleavage and linkage may easily be performed using enzymes, *etc.,* known in the art, but the method is not limited thereto.

As used herein, the term "decrease in expression level compared to its endogenous expression level" refers to inactivation of a particular gene, and specifically, "inactivation" refers to a case where the activity of an enzyme protein originally possessed by a microorganism is weakened compared to its endogenous activity or the activity before modification of the protein, the protein is not expressed, or the protein is expressed but exhibits no activity. Additionally, in the present invention, the term inactivation may be used to refer to all of decrease, deletion, and weakening.

Specifically, the inactivation refers to a concept including a case where the activity of the enzyme itself is weakened compared to that originally possessed by a microorganism due to a modification in the enzyme-encoding polynucleotide, *etc.,* or removed; a case where the level of overall enzyme activity is lower than that of the wild-type strain of the microorganism due to inhibition of expression or inhibition of translation of the gene encoding the enzyme, *etc.,* or removed; a case where all or part of the gene is deleted; and a combination thereof, but the inactivation is not limited thereto.

The inactivation of an enzyme may be achieved by applying various methods well known in the art. Examples of the methods may include 1) a method of substituting the enzyme-encoding gene on the chromosome with a gene mutated to reduce the activity of the enzyme, including the case where the enzyme activity is removed; 2) a method of modifying the expression control sequence of the enzyme-encoding gene on the chromosome; 3) a method of substituting the expression control sequence of the enzyme-encoding gene with a sequence having weak or no activity; 4) a method of deleting all or part of the enzyme-encoding gene on the chromosome; 5) a method of introducing an antisense oligonucleotide (*e.g*., antisense RNA) which binds complementary to a transcript of the gene on the chromosome, thereby inhibiting the translation from the mRNA into the enzyme; 6) a method of artificially incorporating a complementary sequence to the SD sequence into the upstream of the SD sequence of the enzyme-encoding gene, forming a secondary structure, thereby making the attachment of ribosome thereto impossible; 7) a method of incorporating a promoter to the 3' terminus of the open reading frame (ORF) of the corresponding sequence to be transcribed in reverse (reverse transcription engineering (RTE)), *etc.,* and also a combination thereof, but the methods are not particularly not limited thereto.

The gene sequence on the chromosome may be modified by inducing modification in the sequence by deletion, insertion, non-conservative or conservative substitution, or a combination thereof in the gene sequence for further weakening the enzyme activity; or by substituting with a gene sequence which was improved to have weaker activity or a gene sequence which was improved to have no activity, but the method is not limited thereto.

The expression control sequence may be modified by inducing modification in the expression control sequence by deletion, insertion, non-conservative or conservative substitution, or a combination thereof of its nucleic acid sequence so as to further weaken the activity of the expression control sequence; or by substituting with a nucleic acid sequence having much weaker activity. The expression control sequence may include a promoter, an operator sequence, a sequence encoding a ribosome-binding region, and sequences controlling the termination of transcription and translation, but is not limited thereto.

Additionally, the method of deleting all or part of a polynucleotide encoding an enzyme may be performed by substituting the polynucleotide encoding the endogenous target protein within the chromosome with a polynucleotide or marker gene having partial deletion in the nucleic acid sequence using a vector for chromosomal insertion within a cell. In an exemplary embodiment of the method of deleting all or part of a polynucleotide, a method for deleting a polynucleotide by homologous recombination may be used, but the method is not limited thereto.

In a case where the polynucleotide is an aggregate of polynucleotides that can exhibit a function, it may be described as a gene. In the present invention, the term polynucleotide may be used interchangeably with gene.

In the above, the term "part" may vary depending on the kinds of polynucleotides, and it may specifically refer to 1 to 300, more specifically 1 to 100, and even more specifically 1 to 50, but the term is not particularly limited thereto.

In an embodiment of the present invention, strains with an increased concentration of NADPH compared to the control group were selected, and then a yeast strain where *Ald2* gene is inactivated and *Ald6* gene is overexpressed in a PPD yeast cell (PPD,*Δ*ald2::ald6) was prepared from the selected strains so as to confirm whether the genes which affect the NADPH biosynthetic pathway can also affect the growth of PPD yeast cells and PPD production (Example 4). Additionally, a strain where *Zwf1* is further inactivated in the above strain (PPD,*Δ*ald2::ald6,*Δ*zwf1) and a strain where *Zms1* is further overexpressed in the above strain (PPD,*Δ*ald2::ald6,p416_GPD_Zms1) were prepared.

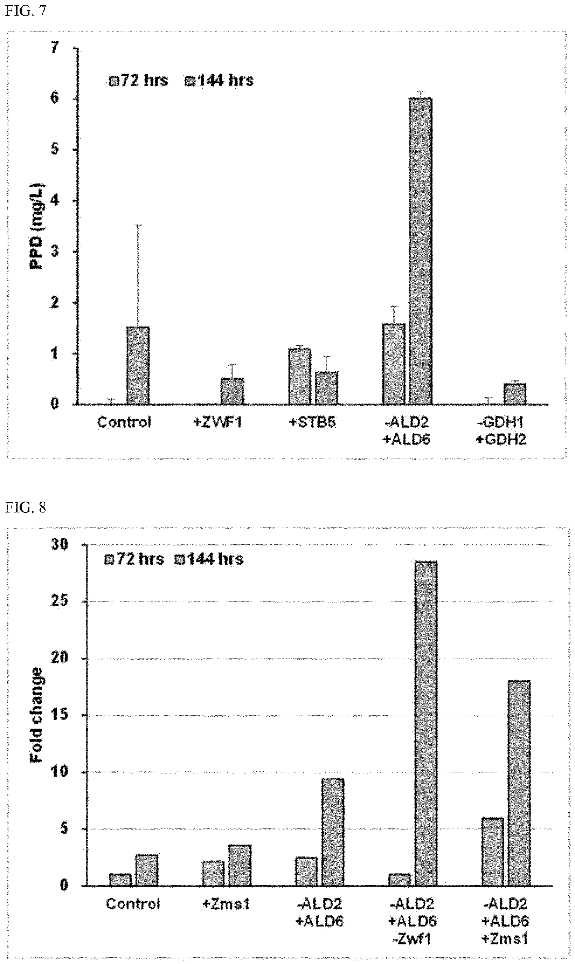

As a result, it was confirmed that the prepared transformed yeast can exhibit higher protopanaxadiol (PPD) productivity compared to the control group. Specifically, it was confirmed that the strain where *Ald2* gene is inactivated and *Ald6* gene is overexpressed can produce about a 4-fold higher protopanaxadiol production compared to the control group, and that in cases where the Zwf1 gene is further inactivated or the Zms1 gene is further overexpressed, where the Zwf1 gene and the Zms1 gene affect the NADPH biosynthetic pathway, the amount of protopanaxadiol production was significantly increased (Table 5 and FIGS. 7 and 8).

From the above results, showing that when the expression levels of the NADPH biosynthesis-related genes (*Ald2, Ald6, Zwf1,* and *Zms1*) were changed by the endogenous expression levels, the amount of production of protopanaxadiol, which is an intermediate product of ginsenoside biosynthesis, was increased, it is expected that the ginsenoside-producing ability of these genes will be improved.

As used herein, the term "yeast for producing ginsenosides" refers to yeast which naturally has a ginsenoside-producing ability; or yeast which does not have a ginsenoside-producing ability in its parent strain but a ginsenoside-producing ability is provided thereto.

Specifically, in the present invention, a ginsenoside-producing microorganism may refer to the wild-type microorganism itself; a microorganism to which a gene related to the external ginsenoside biosynthesis is introduced, thus having a ginsenoside-producing ability; or a microorganism in which a gene related to the mechanism of external NADPH production is inserted, the activity of an endogenous gene thereof is enhanced or inactivated, and its ginsenoside-producing ability is thereby increased.

More specifically, the yeast may be one that belongs to the genus *Saccharomyces,* the genus *Zygosaccharomyces,* the genus *Pichia,* the genus *Kluyveromyces,* the genus *Candida,* the genus *Shizosaccharomyces,* the genus *Issachenkia,* the genus *Yarrowia,* or the genus *Hansenula.*

The yeast belonging to the genus *Saccharomyces* may be, for example, *S. cerevisiae, S. bayanus, S. boulardii, S. bulderi, S. cariocanus, S. cariocus, S. chevalieri, S. dairenensis, S. ellipsoideus, S. eubayanus, S. exiguus, S.florentinus, S. kluyveri, S. martiniae, S. monacensis, S. norbensis, S. paradoxus, S. pastorianus, S. spencerorum, S. turicensis, S. unisporus, S. uvarum,* or *S. zonatus.* More specifically, the yeast may be *Saccharomyces cerevisiae* (*S. cerevisiae*), but the yeast is not limited thereto.

The ginsenoside-producing microorganism may be that where the expression level of *Ald2* gene is decreased compared to its endogenous expression level while the expression level of *Ald6* gene is increased compared to its endogenous expression, and additionally, that where the expression level of *Zwf1* gene is decreased compared to its endogenous expression, but the microorganism is not particularly limited thereto. Additionally, the microorganism capable of producing ginsenosides may be that where the expression level of *Zms1* gene is further increased compared to its endogenous expression, but the microorganism is not limited thereto.

Additionally, the ginsenoside-producing microorganism may be one in which i) HMG-CoA reductase (HMG1), which converts HMG-CoA to mevalonic acid, and ii) Panax ginseng squalene epoxidase (PgSE), for enhancing the mevalonic acid metabolic pathway to increase the biosynthesis of squalene (*i.e.,* an essential precursor for ginsenoside biosynthesis), are modified such that their activities are greater than their endogenous activities, respectively; and iii) Panax ginseng dammarenediol-II synthase (PgDDS), which converts 2,3-oxidosqualene to dammarenediol-II, iv) Panax ginseng cytochrome P450 CYP716A47 (PgPPDS), which converts dammarenediol-II to protopanaxadiol, and v) Panax ginseng NADPH-cytochrome P450 reductase (Panax PgCPR) are modified such that their activities can be introduced, but the modification may not be particularly limited thereto.

As used herein, the term "ginsenoside" refers to a dammarane-type saponin or a derivative thereof, and it has a chemical structure different from those of saponins discovered in other plants. Specifically, ginsenosides may be classified into three different groups based on their aglycone structures: protopanaxadiol (PPD)-type ginsenosides, protopanaxatriol (PPT)-type ginsenosides, and oleanolic acid-type ginsenosides. These three groups may be further classified based on the position and number of sugar moieties attached to the C-3, C-6, and C-20 positions of the rings in the aglycone structure of the compounds by a glycosidic bond. PPDs and PPTs have different hydroxylation patterns. The basic backbone of the oleanolic acid-type ginsenosides is 5-cyclic, and its aglycone is oleanolic acid; ginsenoside Ro is uniquely present in this group. At present, more than 40 kinds of ginsenosides have been isolated, and most are PPD-type ginsenosides. PPD-type ginsenosides include Rb1, Rb2, Rb3, Rc, Rd, gypenoside XVII, Compound O, Compound Mc1, F2, Compound Y, Compound Mc, Rg3, Rh2, and C-K. PPT-type ginsenosides include Re, Rg1, Rf, Rg2, Rh1, *etc.*

In an embodiment, the ginsenosides may be PPD-type ginsenosides, PPT-type ginsenosides, *etc*.; in another embodiment, the PPD, PPT, Ra3, Rb1, Rb2, Rb3, Rc, Rd, Re, Rg1, Rg2, Rg3, Rh1, Rh2, Rs1, C-O, C-Y, C-Mc1, C-Mc, F1, F2, Compound K, Gypenoside XVII, Gypenoside LXXV, Rs2, PPD, Re, Rg1, Rf, F1, Rg2, PPT, Rh1, *etc.* may be used alone or as a mixture; and in still another embodiment, PPD, PPT, compound K, Rb1, Rb2, Rb3, Rc, Rd, Re, F1, F2, Rg1, Rg2, Rg3, Rh1, Rh2, *etc.* may be used alone or as a mixture, and but the ginsenosides are not particularly limited thereto. Specifically, the ginsenosides may be protopanaxadiol-type ginsenosides.

Additionally, the yeast of the present invention may be that where the NADPH-producing ability is increased compared to its endogenous production level.

In another specific embodiment, the present invention provides yeast in which the expression level of the gene involved in the synthesis of ginsenosides is further increased compared to its endogenous expression level.

In still another specific embodiment, the present invention provides yeast in which the gene is at least one selected from the group consisting of Panax ginseng dammarenediol-II synthase (*PgDDS*), Panax ginseng cytochrome P450 CYP716A47 (*PgPPDS*), Panax ginseng NADPH-cytochrome P450 reductase (*PgCPR*), *S. cerevisiae* HMG-CoA reductase (*tHMG1*), and Panax ginseng squalene epoxidase (*PgSE*).

The enzymes related to the ginsenoside biosynthesis metabolic pathway may each have an amino acid sequence having a homology to the amino acid sequences of SEQ ID NOS: 5 to 9, of at least 70%, more specifically at least 80%, and even more specifically at least 90%.

Another aspect of the present invention provides a method for preparing yeast with an improved ginsenoside-producing ability, which includes changing the expression levels of NADPH biosynthesis-related genes compared to their endogenous expression levels.

In a specific embodiment, the present invention provides a method for preparing yeast, in which the expression level of at least one gene selected from the group consisting of *Ald2, Ald6, Zwf1,* and *Zms1* genes, which are NADPH biosynthesis-related genes, is increased compared to its endogenous expression level.

The ginsenoside-producing yeast strain, NADPH biosynthesis-related genes, *Ald2, Ald6, Zwf1,* and *Zms1* genes, endogenous expression level, *etc.* are the same as described above.

In another specific embodiment of the present invention, the ginsenoside-producing yeast strain may be that where the expression level of at least one gene, which is selected from the group consisting of Panax ginseng dammarenediol-II synthase (*PgDDS*), Panax ginseng cytochrome P450 CYP716A47 (*PgPPDS*), Panax ginseng NADPH-cytochrome P450 reductase (*PgCPR*), *S. cerevisiae* HMG-CoA reductase (*tHMG1*), and Panax ginseng squalene epoxidase (*PgSE*), is increased compared to its endogenous expression level.

Still another aspect of the present invention provides a method for producing ginsenosides, which includes culturing the yeast in a medium; and recovering ginsenosides from the yeast or the medium.

In the above method, the cultivation of the yeast may be performed by a known batch culture, continuous culture, fed-batch culture, *etc.,* but the cultivation is not particularly limited thereto. In particular, the culture conditions are not particularly limited, but an optimal pH (*e.g.,* pH 5 to pH 9, specifically pH 6 to pH 8, and most specifically pH 6.8) may be adjusted using a basic compound (*e.g*., sodium hydroxide, potassium hydroxide, or ammonia) or an acidic compound (*e.g*., phosphoric acid or sulfuric acid). An aerobic condition may be maintained by adding oxygen or an oxygen-containing gas mixture to the culture. The culture temperature may be maintained at 20°C to 45°C, and specifically at 25°C to 40°C, and the culturing may be performed for about 10 hours to about 160 hours, but is not limited thereto. The ginsenosides produced by the cultivation may be secreted into the medium or may remain within the cells.

Further, in the culture medium to be used, as a carbon source, sugars and carbohydrates (*e.g.,* glucose, sucrose, lactose, fructose, maltose, molasses, starch, and cellulose), oils and fats (*e.g.,* soybean oil, sunflower seed oil, peanut oil, and coconut oil), fatty acids (*e.g.,* palmitic acid, stearic acid, and linoleic acid), alcohols (*e.g.,* glycerol and ethanol), organic acids (*e.g.,* acetic acid), *etc.* may be used alone or in combination, but the carbon source is not limited thereto. As a nitrogen source, a nitrogen-containing organic compound (*e.g.*, peptone, a yeast extract, a meat extract, a malt extract, a corn steep liquor, soybean meal, and urea) or an inorganic compound (*e.g.,* ammonium sulfate, ammonium chloride, ammonium phosphate, ammonium carbonate, and ammonium nitrate), *etc.* may be used alone or in combination, but the nitrogen source is not limited thereto. As a phosphorus source, potassium dihydrogen phosphate, dipotassium hydrogen phosphate, a sodium-containing salt corresponding thereto, *etc.* may be used alone or in combination, but the phosphorus source is not limited thereto. The medium may also include essential growth-promoting materials such as other metal salts (*e.g.,* magnesium sulfate or iron sulfate), amino acids, and vitamins.

The above method may further include recovering produced ginsenosides. This recovery step may be a step of recovery from the cultured cells or a supernatant thereof, and an appropriate process for recovery may be selected by those skilled in the art.

With regard to the method of recovering the ginsenosides produced during the cultivation of the present invention, the target products may be collected from the culture using an appropriate method known in the art according to the cultivation method (*e.g*., batch culture, continuous culture, fed-batch culture, *etc.*). For example, centrifugation, filtration, anion exchange chromatography, crystallization, HPLC, *etc.* may be used, and the desired ginsenoside may be recovered from the medium or microorganism using an appropriate method known in the art. The method of recovering ginsenosides may further include a step of purification.

### Advantageous Effects of the Invention

The ginsenoside-producing yeast of the present invention, in which the expression levels of the NADPH biosynthesis-related genes are changed, has an effect of increasing the amount of protopanaxadiol production (*i.e*., an intermediate product of ginsenoside biosynthesis) compared to the existing yeast with a ginsenoside-producing ability.

### Brief Description of Drawings

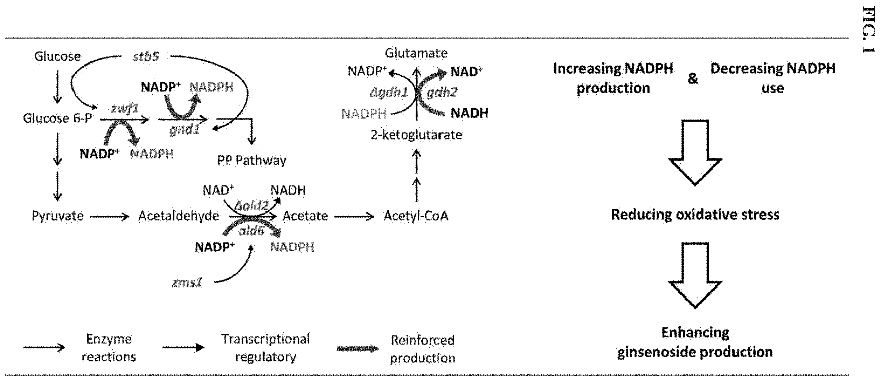

FIG. 1 is a drawing briefly illustrating the increase of ginsenoside production by the enhancement of the NADPH biosynthetic pathway.

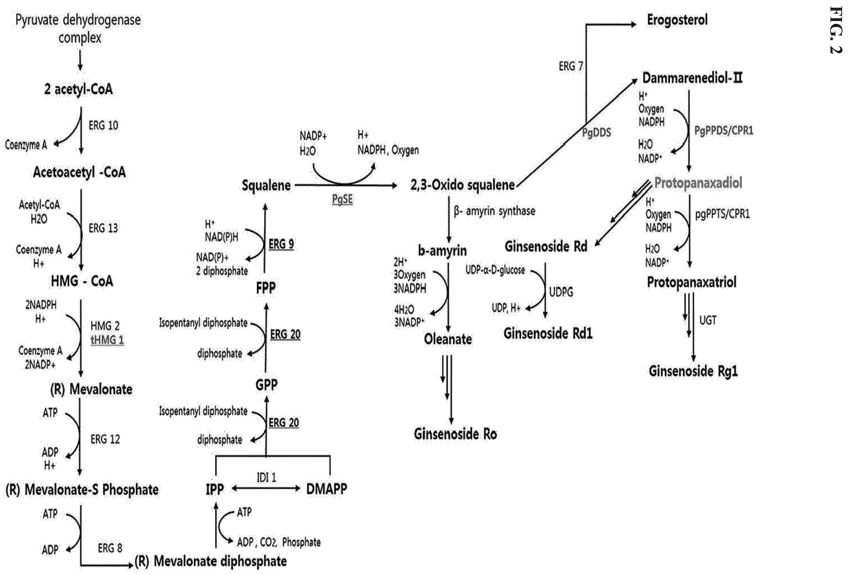

FIG. 2 is a drawing illustrating a metabolic pathway of ginsenoside biosynthesis.
FIG. 3 is a drawing illustrating a vector map of pUC57-URA3Myc, a vector prepared for the inactivation of *Ald2* gene or *Gdh1* gene.
FIG. 4 is a drawing illustrating a vector map of p416_GPD, a vector prepared for the overexpression of *Gnd1* gene, *Gdh2* gene, *Ald6* gene, *Zwf1* gene, or *Stb5* gene.
FIG. 5 is a drawing illustrating a vector map of pUC57GPD-URA3Myc, a vector prepared for the inactivation of *Ald2* gene and overexpression of *Ald6* gene; and for the inactivation of *Gdh1* gene and overexpression of *Gdh2* gene.

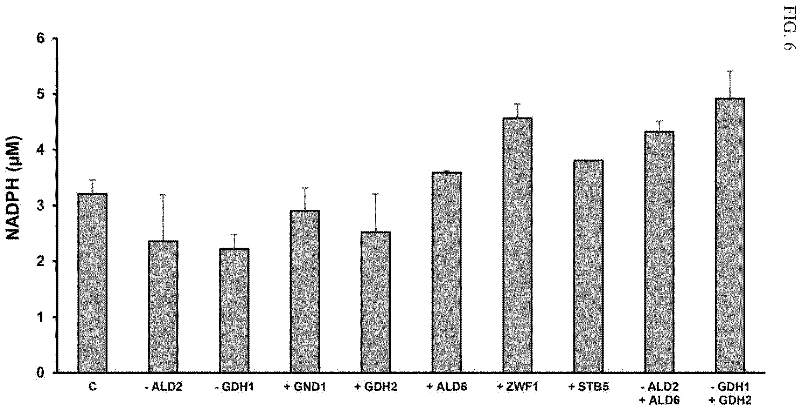

FIG. 6 is a graph illustrating the level of NADPH production in a wild-type yeast cell and a transformed yeast cell, in which C represents *S. cerevisiae* CEN.PK2-1D as a control strain; *-Ald2* represents CEN.PK2-1D,*Δ*ald2; *-Gdh1* represents CEN.PK2-1D,*Δ*Gdh1; +*GND1* represents CEN.PK2-1D,p416_GPD_GND1; +*Gdh2* represents CEN.PK2-1D,p416_GPD_Gdh2; +*Ald6* represents CEN.PK2-1D,p416_GPD_Ald6; +*Zwf1* represents CEN.PK2-1D, p416_GPD_Zwf1; +*STB5* represents CEN.PK2-1D,p416_GPD_STB5; *-Ald2*/+*Ald6* represents CEN.PK2-1D,*Δ*ald2::ald6; and *-Gdh1*/+*Gdh2* represents CEN.PK2-1D,*Δ*Gdh1:: Gdh2.
FIG. 7 is a graph illustrating the level of PPD production in a transformed yeast cell, in which Control represents a PPD strain (*S. cerevisiae* CEN.PK2-1D *Δtrp1::PGPD1 tHMG1* + *PGPD1 PgSE Δleu2::PGPD1 PgDDS* +*PGPD1 PgPPDS* + *PGPD1 PgCPR*); +*Zwf1* represents PPD,p416_GPD_Zwf1; +*STB5* represents PPD,p416_GPD_Stb5; *-Ald2*/+*Ald6* represents PPD,*Δ*ald2::ald6; and *-Gdh1*/+*Gdh2* represents PPD,*Δ*Gdh1::Gdh2.
FIG. 8 is a graph illustrating the level of PPD production in a transformed yeast cell in terms of relative value, in which Control represents a PPD strain (*S. cerevisiae* CEN.PK2-1D *Δtrp1::PGPD1 tHMG1* + *PGPD1 PgSE Δleu2::PGPD1 PgDDS* +*PGPD1 PgPPDS* + *PGPD1 PgCPR);* +*Zms1* represents PPD,p416_GPD_Zms1; *-Ald2*/+*Ald6* represents PPD,*Δ*ald2::ald6; *-Ald2*/+*Ald6*/*-Zwf1* represents PPD,*Δ*ald2::ald6,*Δ*Zwf1; and *-Ald2*/+*Ald6*/+*Zms1* represents PPD,*Δ*ald2::ald6,p416_GPD_Zms1. Each value represents the change in the fold of protopanaxadiol produced in each prepared strain when the production concentration of protopanaxadiol at time 72 hours is set at 1.

### DETAILED DESCRIPTION OF THE INVENTION

Hereinafter, the present invention will be described in more detail with reference to the following Examples and Experimental Examples. However, these Examples and Experimental Examples are for illustrative purposes only and the scope of the invention is not limited by these Examples and Experimental Examples.

### Example 1: Preparation of PPD modified yeast strain

A protopanaxadiol (PPD)-producing yeast strain of the present invention was prepared by i) introducing a ginsenoside biosynthesis metabolic pathway into *Saccharomyces cerevisiae* (*S. cerevisiae*) CEN.PK2-1D wild-type strain [(MATα ura3-52; trp1-289; leu2-3,112; his3Δ 1; MAL2-8; SUC2) and ii) enhancing the mevalonic acid metabolic pathway, which increases biosynthesis of squalene (*i.e.,* an essential precursor for ginsenoside biosynthesis) of the strain, and the prepared strain was named as PPD modified yeast strain(PPD strain).

The genotype of the PPD strain is *S. cerevisiae* CEN.PK2-1D *Δtrp1::P_{GPD1} tHMG1* + *P_{GPD1} PgSE* + *Δleu2::P_{GPD1} PgDDS* + *P_{GPD1} PgPPDS* + *P_{GPD1} PgCPR.*

Genes, which encode ginsenoside biosynthesis enzymes, *i.e*. Panax ginseng dammarenediol-II synthase (PgDDS, SEQ ID NO: 5), Panax ginseng cytochrome P450 CYP716A47 (PgPPDS, SEQ ID NO: 6), and Panax ginseng NADPH-cytochrome P450 reductase (PgCPR, SEQ ID NO: 7); and metabolic pathway enzymes for enhancing the mevalonic acid metabolic pathway tHMG1(*S.cerevisiae* HMG-CoA reductase, SEQ ID NO: 8) and Panax ginseng squalene epoxidase, (PgSE, SEQ ID NO: 9), were each transcribed from GPD1(TDH3), a constitutive high-expression promoter, and expressed.

### Example 2: Preparation of modified strains related to NADPH biosynthetic pathway

### 2-1: Preparation of strain where Ald2 gene or Gdh1 gene is inactivated

To examine whether the inactivation of the *Ald2* gene or *Gdh1* gene, which are genes related to NADPH biosynthesis or consumption pathway, in the PPD modified yeast strain may be involved in the growth of the above yeast strain and PPD-producing ability, modified yeast strains where the *Ald2* gene or *Gdh1* gene was inactivated were prepared.

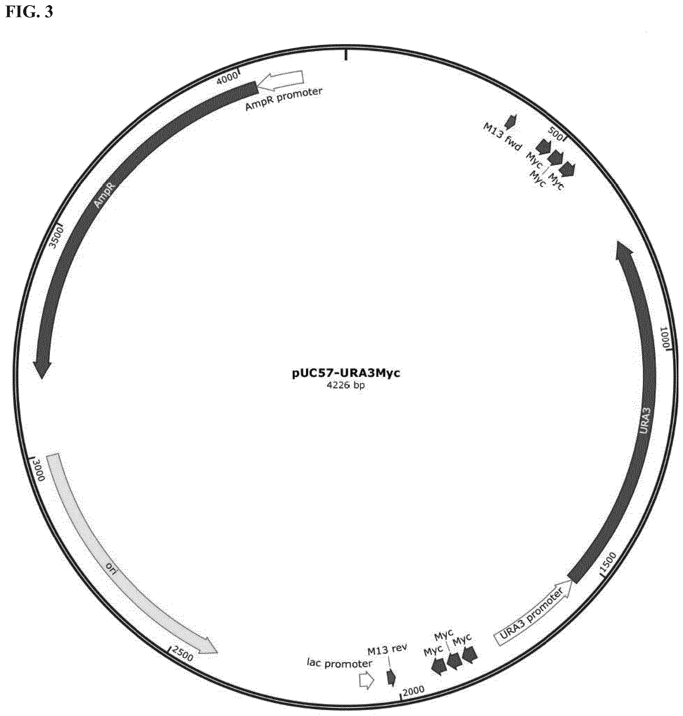

To inactivate the *Ald2* gene on the genome, a cassette that inactivates the *Ald2* gene was prepared by performing PCR using the previously prepared pUC57-URA3Myc vector (Ju Young Lee et al., (2015) Biotechnol. Bioeng., 112, 751-758.) as a template (FIG. 3 and SEQ ID NO: 18) and Del_Ald2_F and Del_Ald2_R, the homologous recombinant sequences of Ald2 gene region on the genome, as primers. The prepared inactivation cassette was transformed into the cells of each wild-type yeast strain of *S. cerevisiae* CEN.PK2-1D. The transformation was performed by conventional heat shock transformation. After the transformation, the cells were cultured in a uracil drop-out medium so that the *Ald2* gene on the genome was able to be substituted with the cassette containing URA3.

The inactivity of the *Ald2* gene in the obtained strain was confirmed by performing PCR using the genomic DNA of the above cells as a template and a primer set of Ald2_conf_F and Ald2_conf_R, and the strain was named as CEN.PK2-1D, *Δ*ald2. In the same manner, a *Gdh1* inactivation cassette was prepared using a primer set of Del_Gdh1_F and Del_Gdh1_R, and the inactivity of the *Gdh1* gene was confirmed using a primer set of Gdh1_conf_F and Gdh1_conf_R primers, and the strain was named as a CEN.PK2-1D, *Δ*Gdh1. The primers used above are shown in Table 1 below.

**Table 1**

| **Name** | **Primer Sequence (5'→3')** | **SEQ ID NO** |
|---|---|---|
| Del_Ald2_F | TTACATTGCATGTCCATCAAAAACAATCGTGAAAATA AGCCAAAAGAAAACCAGTCACGACGTTGTAAAA | 10 |
| Del_Ald2_R | CTGCAACATCCCACTCCTTCTTTGCAGTTTCTTTAAAC TTTTCAACAAACAGGTTTCCCGACTGGAAAGC | 11 |
| Ald2_conf_F | TTACATTGCATGTCCATCAAAAACA | 12 |
| Ald2_conf_R | CTGCAACATCCCACTCCTTC | 13 |
| Del_Gdh1_F | ACTATCGCATTATTCTAATATAACAGTTAGGAGACCA AAAAGAAAAAGAACCAGTCACGACGTTGTAAAA | 14 |
| Del_Gdh1_R | GACGGCAATAGCTTCTGGAGTGGAACCCATGTTGGA ACCTTCGGCAATAAAGGTTTCCCGACTGGAAAGC | 15 |
| Gdh1_conf_ F | CAGTTAGGAGACCAAAAAGAAAAAGAA | 16 |
| Gdh1_conf_ R | GACGGCAATAGCTTCTGGAG | 17 |

### 2-2: Preparation of strain overexpressing Gnd1, Gdh2, Ald6, Zwf1, or Stb5

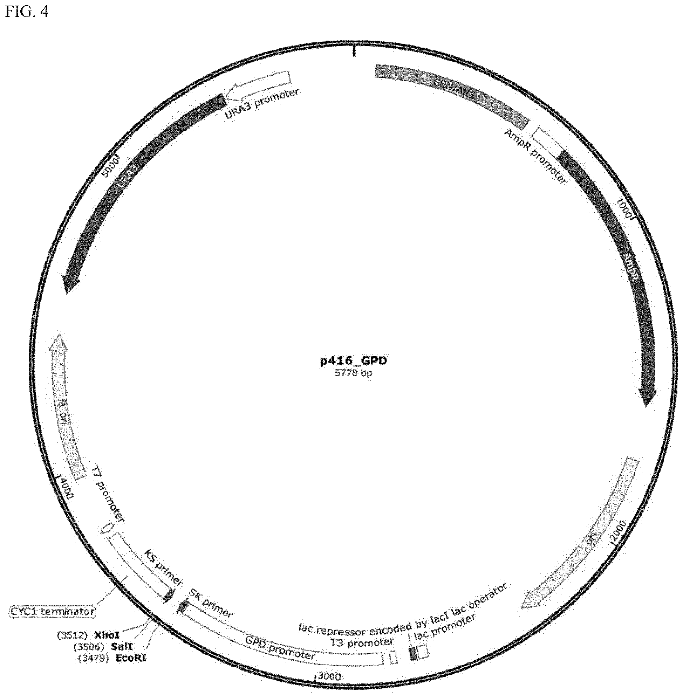

For the overexpression of the *Gnd1* gene, the *Gnd1* gene was amplified from the genomic DNA of *S. cerevisiae* CEN.PK2-1D by performing PCR using a primer set of Gnd1_F and Gnd1_R, and the amplified products were digested with *Eco*RI and *Xho*I*,* and ligated to the p416_GPD vector (FIG. 4 and SEQ ID NO: 29), which was also digested with *Eco*RI and *Xho*I*,* and thereby the p416_GPD_Gnd1 vector was prepared.

For the overexpression of the *Gdh2* gene, the *Gdh2* gene was amplified from the genomic DNA of *S. cerevisiae* CEN.PK2-1D by performing PCR using a primer set of Gdh2_F and Gdh2_R, and the amplified products were digested with *Xba*I and *Sma*I*,* and ligated to the p416_GPD vector, which was also digested with *Xba*I and *Sma*I*,* and thereby the p416_GPD_Gdh2 vector was prepared.

For the overexpression of the *Ald6* gene, the *Ald6* gene was amplified from the genomic DNA of *S. cerevisiae* CEN.PK2-1D by performing PCR using a primer set of Ald6_F and Ald6_R, and the amplified products were digested with *Bam*HI and *Xho*I*,* and ligated to the p416_GPD vector, which was also digested with *Bam*HI and *Xho*I*,* and thereby the p416_GPD_Ald vector was prepared.

For the overexpression of the *Zwf1* gene, the *Zwf1* gene was amplified from the genomic DNA of *S. cerevisiae* CEN.PK2-1D by performing PCR using a primer set of *Zwf1_*F and *Zwf1_*R, and the amplified products were digested with *Eco*RI and *Xho*I*,* and ligated to the p416_GPD vector, which was also digested with *Eco*RI and *Xho*I*,* and thereby the p416_GPD_Zwf1 vector was prepared.

For the overexpression of the *Stb5* gene, the *Stb5* gene was amplified from the genomic DNA of *S. cerevisiae* CEN.PK2-1D by performing PCR using a primer set of Stb5_F and Stb5_R, and the amplified products were digested with *Eco*RI and *Sal*I, and ligated to the p416_GPD vector, which was also digested with *Eco*RI and *Sal*I, and thereby the p416_GPD_Stb5 vector was prepared.

The primers used above are shown in Table 2 below.

**Table 2**

| **Name** | **Primer Sequence (5'→3')** | **SEQ ID NO** |
|---|---|---|
| Gnd1_F | GGAATTCATGTCTGCTGATTTCGGTTT | 19 |
| Gnd1_R | CCGCTCGAGTTAAGCTTGGTATGTAGAGGAAGAA | 20 |
| Gdh2_F | GCTCTAGAATGCTTTTTGATAACAAAAATCGCGG | 21 |
| Gdh2_R | TCCCCCGGGTCAAGCACTTGCCTCCGCTT | 22 |
| Ald6_F | CGGGATCCATGACTAAGCTACACTTTGACACTGC | 23 |
| Ald6_R | CCGCTCGAGTTACAACTTAATTCTGACAGCTTTTACTTCAG | 24 |
| Zwf1_F | GGAATTCATGAGTGAAGGCCCCGTCAA | 25 |
| Zwf1_R | CCGCTCGAGCTAATTATCCTTCGTATCTTCTGGC | 26 |
| Stb5_F | GGAATTCATGGATGGTCCCAATTTTGCAC | 27 |
| Stb5_R | ACGCGTCGACTCATACAAGTTTATCAACCCAAGAGACG | 28 |

For the overexpression of the *Gnd1* gene, *Gdh2* gene, *Ald6* gene, *Zwfl* gene, or *Stb5* gene, the p416_GPD_Gnd1 vector, p416_GPD_Gdh2 vector, p416_GPD_Ald6 vector, p416_GPD_Zwf1 vector, or p416_GPD_Stb5 vector prepared above was transformed into each wild-type yeast strain of *S. cerevisiae* CEN.PK2-1D, respectively. The transformation was performed by conventional heat shock transformation, and the cells were cultured in a uracil drop-out medium so that only those strains where a vector containing a particular gene to be a subject for overexpression and URA3 were able to grow.

As a result, the prepared strains were named as CEN.PK2-1D+Gnd1, CEN.PK2-1D+Gdh2, CEN.PK2-1D+Ald6, CEN.PK2-1D+Zwf1, and CEN.PK2-1D+Stb5, respectively.

### 2-3: Preparation of strains where Ald2 is inactivated and Ald6 is overexpressed, and strains where Gdh1 is inactivated and Gdh2 is overexpressed

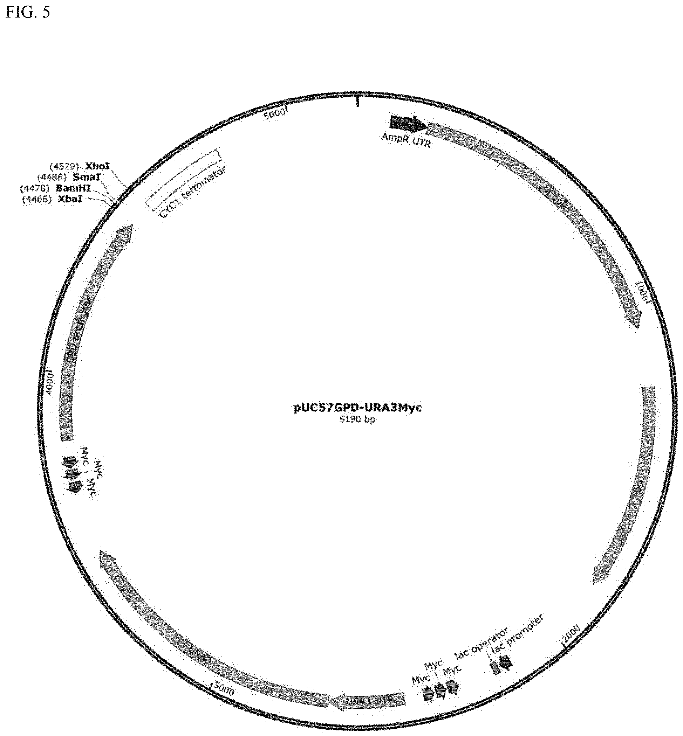

For the inactivation of the *Ald2* gene and overexpression of the *Ald6* gene, the *Ald6* gene was amplified from the genomic DNA of *S. cerevisiae* CEN.PK2-1D by performing PCR using a primer set of Ald6_F and Ald6_R (Table 2) and the amplified products were digested with *Bam*HI and *Xho*I*,* and ligated to the pUC57GPD-URA3Myc vector (Ju Young Lee et al., (2015) Biotechnol. Bioeng., 112, 751 to 758), which was also digested with *Bam*HI and *Xho*I, and thereby the pUC57GPD-URA3Myc_Ald6 vector was prepared (FIG. 5 and SEQ ID NO: 30). Furthermore, for the inactivation of the *Ald2* gene and overexpression of the *Ald6* gene, a cassette that substitutes the *Ald2* gene with the *Ald6* gene was prepared by performing PCR using the above-prepared pUC57GPD-URA3Myc_Ald6 vector as a template and Del_Ald2_F and Del_Ald2_R, the homologous recombinant sequences of *Ald2* gene region on the genome, as primers.

For the inactivation of the *Gdh1* gene and overexpression of *Gdh2* gene, the pUC57GPD-URA3Myc_Gdh2 vector was prepared in the same manner as in Example 2-2 by performing PCR using the primer set Gdh2_F and Gdh2_R (Table 2) and ligation by digestion with *Xba*I and *Sma*I*.* Furthermore, for the inactivation of the *Gdh1* gene and overexpression of *Gdh2* gene, a cassette that substitutes the *Gdh1* gene with the *Gdh2* gene was prepared by performing PCR using the above-prepared pUC57GPD-URA3Myc_Gdh2 vector as a template and Del_Gdh1_F and Del_ Gdh1_R, the homologous recombinant sequences of *Gdh1* gene region on the genome, as primers.

The prepared cassette was transformed into the cells of each wild-type yeast strain of *S. cerevisiae* CEN.PK2-1D. The transformation was performed by conventional heat shock transformation. After the transformation, the cells were cultured in a uracil drop-out medium so that the *Ald2* gene or *Gdh1* gene on the genome was able to be substituted with the cassette containing URA3.

The substitution of the *Ald2* gene with *Ald6* gene and the substitution of *Gdh1* gene with *Gdh2* gene in the strain were confirmed by performing PCR using the genomic DNA of the above cells as a template and a primer set of Ald2_conf_F and Ald2_conf_R and a primer set of Gdh1_conf_F and Gdh1_conf_R, respectively.

As a result, the prepared strains were named as CEN.PK2-1D,*Δ*ald2::ald6 and CEN.PK2-1D,*Δ*Gdh1::Gdh2, respectively.

### Example 3: Confirmation of amount of NADPH production in wild-type yeast and transformed yeast

A wild-type yeast strain, *S. cerevisiae* CEN.PK2-1D, and transformed yeast strains therefrom were inoculated into 50 mL of minimal uracil drop-out media containing 2% glucose such that the absorbance at OD₆₀₀ became 0.5, and cultured while stirring at 30°C at a rate of 250 rpm under aerobic conditions for 24 hours.

At the time of terminating the culture, the amount of nicotinamide adenine dinucleotide phosphate (NAPDH) in the cell was analyzed using the EnzyChrom™ NADP⁺/NADPH assay kit (ECNP-100). The accompanying procedures of the analysis method were adjusted to be suitable for the experimental conditions as follows.

Specifically, the cell culture containing 10 OD (about 8 × 10⁹ cells) based on the OD₆₀₀ measurement using a spectrophotometer was separated by a centrifuge, the supernatant was discarded, and the cells in the form of a pellet were collected, washed with a cold PBS buffer, and mixed with an NADPH extraction buffer. The resultant was heated at 60°C for 5 minutes, and an assay buffer (20 µL) and an NADPH extraction buffer (100 µL) were added thereto and mixed well. The mixed solution was centrifuged at 14,000 rpm for 5 minutes using a centrifuge, and the sample values set at 565 nm were measured using only the supernatant by a spectrophotometer. The measured values were substituted into a calibration curve and the concentration values of NADPH were obtained. The concentration values of NADPH after 24 hours of culture are shown in FIG. 8 below.

Specifically, as shown in FIG. 8, it was confirmed that the NADPH concentration in the cells of each of the strain with overexpression of *Zwf1* gene (CEN.PK2-1D+Zwf1), the strain with overexpression of *Stb5* gene (CEN.PK2-1D+STB5), the strain with inactivation of *Ald2* gene and overexpression of *Ald6* gene (CEN.PK2-1D, *Δ*ald2::ald6), and the strain with inactivation of *Gdh1* gene and overexpression of *Gdh2* gene (CEN.PK2-1D, *Δ*Gdh1::Gdh2) were shown to be higher than that of the control group.

### Example 4: Confirmation of growth and amount of PPD production of transformed modified strains

The strains where the cellular concentration of NADH was increased compared to that of the control group were selected in Example 3, and the effects of the genes from these strains, which affect the NADPH biosynthetic pathway, on the growth of PPD yeast cells and their PPD production were examined.

### 4-1: Preparation of strains where Zwf1 or Stb5 is overexpressed in PPD yeast cells

First, the *Zwf1* gene and *Stb5* gene were introduced into PPD yeast cells and overexpressed therein, and the effect of the overexpression of these genes on the growth of PPD yeast cells and their PPD production were examined.

Specifically, for overexpression of the *Zwf1* gene or *Stb5* gene in PPD yeast cells, and the p416_GPD_Zwf1 vector and the p416_GPD_Stb5 vector prepared in Example 1 were introduced into the PPD yeast strain, respectively. The transformation was performed by conventional heat shock transformation, and the cells were cultured in a uracil drop-out medium so that only those strains where the p416_GPD_Zwf1 vector or the p416_GPD_Stb5 vector containing URA3 was introduced were able to grow.

As a result, the prepared strains were named as PPD, p416_GPD_Zwf1 and PPD, p416_GPD_Stb5, respectively.

### 4-2: Preparation of yeast strains where Ald2 is inactivated and Ald6 is overexpressed, and strains where Gdh1 is inactivated and Gdh2 is overexpressed in PPD yeast cells

In PPD yeast cells, the expression of the *Ald2* gene was inhibited by substituting it with *Ald6* gene while simultaneously overexpressing the *Ald6* gene under the strong GPD promoter, and the effect of the overexpression of the *Ald6* gene on the growth of PPD yeast cells and PPD production were examined. In the same manner, *Gdh1* gene was inhibited by substituting it with *Gdh2* gene and then the *Gdh2* gene was overexpressed, and the effect of the overexpression of the *Gdh2* gene on the growth of PPD yeast cells and PPD production were examined.

Specifically, for the inactivation of the *Ald2* gene and overexpression of *Ald6* gene, and the inactivation of the *Gdh1* gene and overexpression of the *Gdh2* gene in PPD yeast cells, a DNA cassette for homologous recombination was obtained in the same manner as described above, using pUC57GPD-URA3Myc_Ald6 and pUC57GPD-URA3Myc_Gdh2. The prepared cassette was introduced into each PPD yeast strain. The transformation was performed by conventional heat shock transformation. After the transformation, the cells were cultured in a uracil drop-out medium so that only the *Ald2* gene or *Gdh1* gene on the genome was able to be substituted with the cassette containing URA3. The substitution of the *Ald2* gene with *Ald6* gene and the substitution of *Gdh1* gene with *Gdh2* gene in the obtained strains was confirmed by performing PCR using the genomic DNA of the above cells as a template and a primer set of Ald2_conf_F and Ald2_conf_R and a primer set of Gdh1_conf_F and Gdh1_conf_R, respectively.

As a result, the prepared strains were named as PPD,*Δ*ald2::ald6 and PPD,*Δ*Gdh1::Gdh2, respectively.

### 4-3: Preparation of strains where Zwf1 is inactivated in PPD yeast cells

In the strains prepared in Example 4-2, the *Zwfl* gene was further inactivated so as to control the PP pathway, and the effect of the detoured carbon-flux due to the inactivation of the *Zwf1* gene on the growth of PPD yeast cells and their PPD production were examined.

Specifically, a cassette that inactivates the *Zwf1* gene was obtained by performing PCR using the pUC57-URA3Myc vector (Ju Young Lee et al., (2015) Biotechnol. Bioeng., 112, 751 to 758) prepared above as a template and Del_Zwf1_F and Del_Zwf1_R, the homologous recombinant sequences of *Zwf1* gene region on the genome, as primers. The prepared inactivation cassette was transformed into the yeast strain of PPD,*Δ*ald2::ald6. The transformation was performed by conventional heat shock transformation. After the transformation, the cells were cultured in a uracil drop-out medium so that the *Zwf1* gene on the genome was able to be substituted with the cassette containing URA3.

The inactivity of the *Zwf1* gene in the obtained strain was confirmed by performing PCR using the genomic DNA of the above cells as a template and a primer set of Zwf1_conf_F and Zwf1_conf_R, and the strain was named as PPD,*Δ*ald2::ald6,*Δ*zwf1. The primers used above are shown in Table 3 below.

**Table 3**

| **Name** | **Primer Sequence (5'→3')** | **SEQ ID NO** |
|---|---|---|
| Del_Zwf1_F | TATAGACAGAAAGAGTAAATCCAATAGAATAGAAAA CCACATAAGGCAAGCCAGTCACGACGTTGTAAAA | 34 |
| Del_Zwf1_R | CCTCCCAACGCTCGTTTTCGATGTTGAAAGTCATTGCT GCAAAAGTGACAAGGTTTCCCGACTGGAAAGC | 35 |
| Zwf1_conf_F | AGAATAGAAAACCACATAAGGCAAG | 36 |
| Zwf1_conf_R | CCTCCCAACGCTCGTTTTCG | 37 |

### 4-4: Preparation of vector for overexpression of Zms1 in PPD yeast cells

Additionally, in the strains prepared in Example 4-2, *Zms1* gene was overexpressed and the effects of the overexpression of the *Zms1* gene on the growth of PPD yeast cells and PPD production were examined.

Specifically, for the overexpression of the *Zms1* gene, the *Zms1* gene was amplified by performing PCR using the genomic DNA of *S. cerevisiae* CEN.PK2-1D and a primer set of Zms1_F and Zms1_R. The amplified PCR products were digested with *Xma*I and *Xho*I and then ligated to the p416_GPD vector, which was also digested with *Xma*I and *Xho*I*,* and thereby the p416_GPD_Zms1 vector was prepared. The primers used above are shown in Table 4 below.

**Table 4**

| **Name** | **Primer Sequence (5'→3')** | **SEQ ID NO** |
|---|---|---|
| Zms1_F | TAGTGGATCCCCCGGGATGTTTGTGAACGGTAATCAAT CTAATTTC | 38 |
| Zms1_R | AATTACATGACTCGAGTTATATTCTAGTGTTTCTTTTTT TCGTAAC | 39 |

For the overexpression of the *Zms1* gene, the p416_GPD_Zms1 vector prepared above was introduced into each of the PPD yeast strain and the PPD,*Δ*ald2::ald6 strain. The transformation was performed by conventional heat shock transformation, and the cells were cultured in a uracil drop-out medium so that only those strains where the p416_GPD_Zms1 vector containing URA3 introduced were able to grow.

As a result, the prepared strains were named as PPD,p416_GPD_Zms1 and PPD,*Δ*ald2::ald6,p416_GPD_Zms1, respectively.

### Example 5: Confirmation of growth of transformed yeast strains and their PPD production

The transformed yeast strains prepared above were inoculated into 50 mL of minimal URA drop-out media containing 2% glucose such that the absorbance at OD₆₀₀ became 0.5, and cultured while stirring at 30°C at a rate of 250 rpm under aerobic conditions for 144 hours. The OD₆₀₀ values of the cells during the culture were measured using a spectrophotometer. The intracellular metabolites (*i.e.,* squalene, 2,3-oxidosqualene, and protopanaxadiol) were analyzed by high performance liquid chromatography (HPLC).

As a result, the cell growth (*i.e.,* the OD₆₀₀ values of the cell culture) and the concentration of each intracellular metabolite are shown in the following Table 5, and FIGS. 7 and 8.

**Table 5**

| | **OD₆₀₀** | | **Protopanaxadiol (mg/L)** | |
|---|---|---|---|---|
| | **72 h** | **144 h** | **72 h** | **144 h** |
| **Control** | 26.75 | 24.62 | 0 | 1.52 |
| **+Zwf1** | 26.03 | 22.91 | 0.03 | 0.51 |
| **+STB5** | 19.70 | 20.80 | 1.09 | 0.64 |
| **-Ald2+Ald6** | 13.19 | 11.88 | 1.58 | 6.01 |
| **-Gdh1+Gdh2** | 24.82 | 24.08 | 0 | 0.40 |

Specifically, referring to the results of FIG. 7, it was confirmed that the amounts of PPD production in the strains where the *Ald2* gene was inactivated and the *Ald6* gene was overexpressed, after 4 hours of culture, were about 4-fold greater compared to that of the control group. Additionally, it was confirmed that in the strains where the *Zwf1* gene, which affects the NADPH biosynthetic pathway, was further inactivated or where the *Zms1* gene was further overexpressed, the amount of protopanaxadiol production was significantly increased, and in particular, in the strains where the *Zwf1* gene was further inactivated, the amount of protopanaxadiol production was about 20-fold or greater compared to that of the control group (FIG. 8).

From the above results, it was confirmed that when the expression levels of NADPH biosynthesis-related proteins (Ald2, Ald6, Zwf1, and Zms1) were changed compared to their endogenous expression levels, the amount of the protopanaxadiol production was significantly increased.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. Yeast for producing ginsenosides, wherein the expression levels of NADPH biosynthesis-related genes are changed compared to their endogenous expression levels.

2. The yeast of claim 1, wherein the NADPH biosynthesis-related genes are one or more genes selected from the group consisting of *Ald2, Ald6, Zwf1,* and *Zms1.*

3. The yeast of claim 2, wherein the expression level of *Ald2* is decreased compared to its endogenous expression level and the expression level of *Ald6* is increased compared to its endogenous expression level.

4. The yeast of claim 3, wherein the expression level of *Zwf1* is further decreased compared to its endogenous expression level.

5. The yeast of claim 3, wherein the expression level of *Zms1* is further increased compared to its endogenous expression level.

6. The yeast of claim 1, wherein the NADPH-producing ability is increased compared to its endogenous level.

7. The yeast of claim 1, wherein the expression levels of ginsenoside synthesis-related genes are further increased compared to their endogenous expression levels.

8. The yeast of claim 7, wherein the genes are one or more selected from the group consisting of *PgDDS* (Panax ginseng, dammarenediol-II synthase), *PgPPDS* (Panax ginseng cytochrome P450 CYP716A47), *PgCPR* (Panax ginseng, NADPH-cytochrome P450 reductase), *tHMG1* (*S. cerevisiae* HMG-CoA reductase), and *PgSE* (Panax ginseng, squalene epoxidase).

9. A method for preparing recombinant yeast with an enhanced ginsenoside-producing ability, comprising changing the expression levels of the NADPH biosynthesis-related genes compared to their endogenous expression levels.

10. The method of claim 9, wherein the NADPH biosynthesis-related genes are one or more genes selected from the group consisting of *Ald2, Ald6, Zwf1,* and *Zms1.*

11. The method of claim 9, wherein the expression level of *Ald2* is decreased compared to its endogenous expression level and the expression level of *Ald6* is increased compared to its endogenous expression level.

12. The method of claim 11, wherein the expression level of *Zwf1* is further decreased compared to its endogenous expression level.

13. The method of claim 11, wherein the expression level of *Zms1* is further increased compared to its endogenous expression level.

14. The method of claim 9, wherein the expression levels of one or more genes selected from the group consisting of *PgDDS* (Panax ginseng, dammarenediol-II synthase), *PgPPDS* (Panax ginseng cytochrome P450 CYP716A47), *PgCPR* (Panax ginseng, NADPH-cytochrome P450 reductase), *tHMG1* (*S. cerevisiae* HMG-CoA reductase), and *PgSE* (Panax ginseng, squalene epoxidase) are increased compared to their endogenous expression levels.

15. A method for producing ginsenosides, comprising:
(a) culturing the yeast of any one of claims 1 to 8 in a medium; and
(b) recovering ginsenosides from the yeast or the medium.
